# EUROPEAN PATENT APPLICATION

(11) **EP 1 493 748 A1**
(43) Date of publication of application: **05.01.2005**
(21) Application number: 03745998.9
(22) Date of filing: 11.04.2003
(51) Int. Cl.: C07K 7/08, C07K 1/06, C07K 14/00

(54) **PEPTIDE CHEMICALLY MODIFIED WITH POLYETHYLENE GLYCOL**

(30) Priority: 11.04.2002 JP 2002109734
(71) Applicant: MOCHIDA PHARMACEUTICAL CO., LTD., Shinjuku-ku Tokyo 160-8515 (JP)
(72) Inventor: TAGUCHI, Yasushi, MOCHIDA PHARMACEUTICAL CO., LTD, Tokyo 160-8515 (JP); HAZE, Kyousuke, MOCHIDA PHARMACEUTICAL CO., LTD, Tokyo 160-8515 (JP); KURIYAMA, Shinichi, MOCHIDA PHARMACEUTICAL CO. LTD, Tokyo 160-8515 (JP)
(74) Representative: Grünecker, Kinkeldey, Stockmair & Schwanhäusser Anwaltssozietät
(86) International application number: PCT/JP2003/004614
(87) International publication number: WO 2003/084985

(57) **Abstract**

It is intended to provide a peptide chemically modified with PEG which contains a sequence consisting of 18 amino acids and having a specific structure made up of four planes, i.e., two hydrophobic planes and two hydrophilic planes alternately arranged in an alpha-helix structural model; a complex of the above peptide with a peptide-binding substance; a carrier modified with the peptide chemically modified with PEG as described above; a process for producing the same; and a method of delivering a substance bonded to a carrier modified with the peptide chemically modified with PEG or enclosed therein. The peptide chemically modified with PEG as described above has a high safety and can be easily formulated into a complex with a peptide-binding substance (i.e., having favorable handling properties). The resultant complex has a high solubility and shows an excellent introduction selectivity of the peptide-binding substance into cells. Thus, it is available as a vector achieving a high introduction efficiency without lowering the specific activity by the chemical modification with PEG.

## Description

### TECHNICAL FIELD

This invention relates to a peptide which has been chemically modified with polyethylene glycol (hereinafter abbreviated as "PEG") (hereinafter abbreviated as "peptide chemically modified with PEG"), which peptide includes a sequence of 18 amino acids which is constituted from alternately arranged two hydrophobic sides and two hydrophilic sides in α-helix structural model depicted by Edmundson wheel plots, wherein at least one of the two hydrophilic sides is a positively charged side. This invention also relates to its production method.

This invention also relates to a complex of a peptide chemically modified with PEG and a substance which binds to the peptide, and its production method.

This invention also relates to a carrier modified with a peptide chemically modified with PEG, and its production method.

This invention also relates to a method for delivering a substance to the interior of a cell, wherein the substance is bonded to or incorporated in the carrier which has been modified with the peptide chemically modified with PEG.

### BACKGROUND ART

Entire genome sequences are being determined in an increasing number of pathogenic microorganisms, pathogenic viruses, and human, and various attempts are being made to treat various diseases by using the genetic information obtained. One such attempt is the treatment wherein a DNA, an RNA, or a derivative, a modification, or an analog thereof, namely, a nucleic acid is administered to the interior of the body. Through such administration of the nucleic acid to the body, these treatments attempt to treat the disease by increasing or decreasing the amount of particular gene expressed or the extent of the function of particular physiologically active factor developed, or by producing the physiologically active substance coded by the introduced nucleic acid. In most of such treatment, a vector (an agent for introduction) is used as a means for increasing the efficiency of nucleic acid introduction into the cell.

One typical vector is a viral vector, wherein the infectivity inherent to the virus is utilized. Examples of such viral vectors include retrovirus vector and adenovirus vector, and exemplary applications of such viral vector are introduction of adenosine deaminase gene which is an attempt to treat adenosine deaminase deficiency by gene therapy (Blaese, R.M. et al., Science, Vol. 270, 475 (1995)), and introduction of p53 gene for cancer treatment (Swisher, S.G. et al., J. Natl. Cancer Inst., Vol. 91, 763 (1999)). A viral vector, however, is associated with a considerable risk in terms of its safety despite its excellent efficiency in introducing the nucleic acid to the cell. To be more specific, a viral vector is known to suffer from the risk of emergence of the wild type virus (pathogenic virus) and the risk of inducing a serious immune response by its high antigenicity. In addition, the process of production of a viral vector is an extremely complicated, and production of such viral vector in a commercial scale is generally difficult.

Another typical vector is a liposome vector. This vector utilizes the phenomenon that a charged liposome becomes attached to the cell and the liposome then becomes incorporated in the cell, and the liposome vectors known in the art include liposomes having a nucleic acid incorporated therein, and the liposomes aggregated around a nucleic acid to form a mass. Exemplary applications of the liposome having a nucleic acid incorporated therein include introduction of interferon β gene for treating brain tumor (M. Mizuno et al., Cancer Res., Vol. 50, 7826 (1990)), and exemplary uses of the method wherein a mass is formed by attaching liposomes around the nucleic acid include introduction of gene into a cultivated cell, which is frequently conducted in cell engineering experiment (Felgner, P.L. et al., Proc. Natl. Acad. Sci. U.S.A., Vol. 84, 7413 (1987)). When the liposome vector is the one mainly comprising a natural phospholipid, the liposome vector is by far superior to the viral vector in view of safety. Such liposome vector, however, suffer from the problems of the complicated process of vector production, the complicated process of producing the complex of the vector and the nucleic acid, and the low efficiency in introducing the nucleic acid in the cell. When the liposome is constituted from a synthetic lipid, efficiency of nucleic acid introduction into the cell and handling convenience will be improved. Such liposome vector, however, suffers toxicity development. Also, both liposome vectors need further improvements in the drug preparation because of the poor storability of the complex of the liposome and the nucleic acid.

Another exemplary vector is a peptide vector, and an example of such peptide vector is polylysine and its modified form. This vector utilizes the nature that a positively charged peptide tends to electrostatically bind to the negatively charged nucleic acid, and also to a cell. It has been revealed from various studies that, when the polylysine is used alone as a peptide vector, the oligonucleotide which has been covalently bonded to the polylysine is introduced in the cell (Lemaitre, M. et al., Proc. Natl. Acad. Sci. U.S.A., Vol. 84, 648 (1987)). However, it has also been revealed that modification of the polylysine with a sugar, a glycoprotein, a phospholipid, or the like is required to substantially introduce the oligonucleotide or the plasmid that has been electrostatically bonded to the polylysine in a cell (Wu, G.Y. et al., J. Biol. Chem., Vol. 262, 4429 (1987), Zhou, X. et al., Biochim. Biopys. Acta, Vol. 1065, 8 (1991), Liang, W.W. et al., Biochim. Biopys. Acta, Vol. 1279, 227 (1996)). It should also be noted that the unmodified polylysine exhibits significant toxicity when administered into the body of an animal.

Another exemplary peptide vector is an amphipathic basic peptide having α-helix structure, which has been shown to be a peptide usable alone as a vector for the nucleic acid since it exhibits high efficiency in introducing the nucleic acid due to its structural characters (Niidome, T. et al., J. Biol. Chem., Vol. 272, 15307 (1997)).

This peptide, however, suffers from the drawback that, in the α-helix structural model by Edmundson wheel plots, it exhibits typical two sided structure constituted from the hydrophobic side and the charged side (hydrophilic side), and when the proportion of the hydrophobic side is increased in order to maintain the ability of introducing the nucleic acid into the cell, the solubility of the peptide in water is decreased.

In this respect, if the proportion of the hydrophobic side were reduced in order to improve the solubility in water, ability of introducing the nucleic acid into the cell would be compensated. In addition, the proportion of the hydrophilic side, which is the charged side, of the peptide is small, and hydrophilicity of the peptide is lost once the nucleic acid has become electrostatically bound to the peptide, and the complex of the peptide and the nucleic acid becomes hard to be soluble. As a consequence, the peptide suffers from the drawback that aggregates are liable to be formed, and such aggregate formation is a substantial problem. Furthermore, the peptide will be highly toxic when it is administered to the interior of the animal body due to the high tendency of the aggregate formation in serum.

As described above, despite the excellent general handling convenience of the peptide vector that it is capable of forming a complex merely by mixing with the nucleic acid, the peptide vector suffers from the drawback that it has a high tendency of forming aggregates especially in blood, and in addition, that the complex of the peptide vector and the nucleic acid exhibits a low solubility, and hence, a high toxicity. Therefore, the peptide vector has drawbacks to practical use.

In addition, all of the above-described vectors had no selectivity for the cell to which it is to be introduced, and the nucleic acid was introduced not only to the cell wherein the introduction of the nucleic acid was intended but also to the cell wherein the introduction of the nucleic acid was not at all intended. Such non-selective introduction has been associated with the risk of developing side effects.

By the way, phosphatidyl serine and phosphatidyl ethanolamine are aminophospholipids which are constituents of the lipid bilayer constituting the cell surface layer, and these aminophospholipids are phospholipids whose ratio of the content in the outer layer to the content in the inner layer of the lipid bilayer varies according to the conditions of the cell.

To be more specific, phosphatidyl serine and phosphatidyl ethanolamine are phospholipids whose content in the outer layer of the lipid bilayer of the cell membrane increases in relation to the content in the inner layer when the cell receives some stimulus as typically found in the cell in the site where blood coagulation reaction is proceeding (Alan J. Schroit et al., Biochim. Biophys. Acta, Vol. 1071, 313 (1991)). Proportion of these phospholipids in the outer layer of the lipid bilayer is also believed to increase in the cells at the site where inflammation or cell activation and/or injury, apoptosis, or other so-called immunoresponsive reaction caused by immunocompetent cell has taken place, the site where cells have become malignantly transformed through the progress of abnormal cell division, the site where the cells constituting the blood vessel have been injured by blood coagulation or by the progress of arterial sclerosis, the site where a cytotoxic reaction induced by active oxygen is in progress, and the site where cell activation and/or cell injury by protease is in progress, and to be more specific, in the injured, denatured, or activated cell, namely, in the so-called abnormal cell. Phosphatidyl serine is also known to be a phospholipid which is found in the granule, and which becomes translocated to the cell surface as a content of the granule in the course of degranulation in the cell (for example, mast cell or basophil) experiencing an allergic reaction (degranulation) caused by the binding of an allergen to the IgE antibody on the cell surface (Martin, S. et al., Int. Arch. Allergy and Immunol., Vol. 123, 249 (2000)).

Recent studies report that, even in the case of a cell which has been administered with an apoptosis-inducing substance (for example, an anticancer drug) or a cell which has been irradiated with radiation, and even if the cell had experienced signal transduction wherein p53 or other apoptosis-related gene had been involved, apoptosis does not take place if the cell is drug resistant (anticancer drug-resistant cancer cell etc.), and the drug resistant cell survives after DNA repair, and in the course of such DNA repair, phosphatidyl serine is translocated to the cell surface layer (Geske, FJ. et al., Cell Death Differ., Vol. 8, 182 (2001)).

Many studies have revealed that modification of a physiologically active peptide or polypeptide with PEG is an effective way of promoting in vivo functioning of the peptide or the polypeptide. Such modification with the PEG presumably results in an increased hydrophilicity of the peptide or the polypeptide which in turn results in the reduced in vivo interaction with proteins as well as less likeliness of being recognized by reticuloendothelial system, and hence, reduced possibility of being captured by macrophage and the like. As a consequence, in vivo dynamics is improved to enable maintenance of the in vivo physiological activity. Exemplary uses of the PEG modification include the case of PEG-ADA which is adenosine deaminase (ADA) modified with PEG whose clinical effectiveness has been proven in the treatment of ADA deficiency (Hershfield M.S. et al., N. Engl. J. Med., 316, 589 (1987)) and the case of the modification of the interferon (IFN) with PEG which has resulted in the enhancement of the in vivo antiviral action (Perry CM et al., Drugs, 61, 2263 (2001)).

Usefulness of the PEG modification has been revealed not only for the physiologically active peptide and polypeptide but also for a drug carrier used for the purpose of drug delivery (Maruyama, K., Nihon Rinsho, 80, 632 (1998)). For example, liposome modification with PEG has been revealed to be effective in extending the life in the body and also in reducing antigenicity due to the reduced recognizability by the reticuloendothelial system, as in the case of the PEG-modified peptide and polypeptide. Exemplary such uses of the modification of the drug carrier with PEG include the case wherein a liposome having an anticancer drug doxorubicin incorporated therein is modified with PEG, the modification resulting in an increased drug effectivity compared to the unmodified liposome (Sakakibara, T. et al., Cancer Res., 56, 3743 (1996)) .

Attempts have also been made to impart site targetability to the PEG-modified carrier in order to reduce the side effects and improve the drug effectivity. An exemplary such attempt is the modification of a PEG-modified carrier further with a PEG-modified antibody (Maruyama K. et al., Biochim. Biophys. Acta, 1234, 74 (1995)).

Examples other than the liposome include PEG-modified polylysine used in the introduction of a gene (Lee, M. et al., Mol. Ther., 4, 339 (2001)).

Despite the high toxicity of the polylysine, it has been known that the toxicity of polylysine can be reduced by the modification with PEG.

While the PEG modification is a useful technique as described above, it has the drawback that the peptide or the polypeptide after the modification exhibits reduced specific activity, which is caused by the PEG modification of the active center. For example, specific activity of arginase has been shown to reduce to 65% when it is modified with PEG (Savoca, K.V. et al., Biochim. Biophys. Acta, 578, 47 (1979)).

Accordingly, usefulness of the PEG modification is expected to be improved if PEG modification can be accomplished without reducing the specific activity.

When a PEG-modified antibody is used as a means for imparting the site targetability to the PEG-modified carrier, there is a problem in that an antibody is susceptible to steric hindrance due to the PEG molecule and the effect is less likely to be realized. Accordingly, development of a more effective means for imparting site targetability is awaited.

In view of such situation, an object of the present invention is to provide a novel peptide chemically modified with PEG which is highly safe; which can easily make a complex with a substance which binds to the peptide (enjoying excellent handling convenience), the thus produced complex exhibits excellent solubility; which can serve a vector with high selective and efficient introduction of the substance which binds to the peptide into a cell; and whose specific activity has not been compensated by the chemical modification with the PEG; as well as its production method.

Another object of the present invention is to provide a complex of the peptide chemically modified with PEG and a substance which binds to the peptide, and its production method.

A further object of the present invention is to provide a peptide chemically modified with PEG which can impart a site targetability to a carrier having a drug bound thereto or incorporated therein; a carrier modified with such peptide; and their production method.

A still further object of the present invention is to provide a method for delivering a substance bound to or incorporated in the carrier, which has been modified with the peptide chemically modified with PEG, into the cell.

### DISCLOSURE OF THE INVENTION

The inventors of the present invention have made an intensive study, and found that:
(1) a peptide which binds to the substance that binds to the peptide (the peptide used in the present invention and the peptide chemically modified with PEG of the present invention as will be described below) (herein abbreviated as the "peptide-binding substance") and which has affinity for a certain phospholipid can be a vector for the peptide-binding substance which exhibits high efficiency in introducing the peptide-binding substance into a cell, low toxicity, and an excellent solubility;
(2) a peptide which has a particular structural character defined by the amino acid sequence exhibits high localizability on a particular phospholipid, and hence, high selectivity in introducing the peptide-binding substance into a particular cell;
(3) efficiency in introducing the peptide-binding substance into the cell can be improved by chemically modifying the peptide with PEG;
(4) the peptide chemically modified with PEG obtained by modifying the peptide with PEG can be used as an element for imparting site targetability to a carrier having a drug bound thereto or incorporated therein; and
(5) the substance which has been bound to or incorporated in the carrier which is modified with the peptide chemically modified with PEG is efficiently delivered to a particular cell.
The present invention has been completed on the basis of such findings.

Accordingly, the present invention provides a peptide chemically modified with PEG according to (1) to (9) or (21), below; a complex of the peptide chemically modified with PEG and the peptide-binding substance according to (10) to (19) or (24), below; a method for producing the peptide or the complex according to (20), (22), or (23), below; a carrier which is modified with the peptide chemically modified with PEG according to (25) or (27), below; a method for producing the carrier according to (26), below; and a method for delivering a substance according to (28), below.

The first aspect of the present invention is as follows:
(1) A peptide chemically modified with polyethylene glycol (PEG), including a sequence of 18 amino acids, wherein
   said sequence of 18 amino acids is constituted from alternately arranged two hydrophobic sides and two hydrophilic sides in α-helix structural model depicted by Edmundson wheel plots,
   one of said hydrophobic sides comprises 5 to 7 amino acids and 80 mole% or more of this side comprises hydrophobic amino acids,
   one of said hydrophilic sides comprises 5 or 6 amino acids, and 80 mole% or more of this side comprises hydrophilic amino acids, and 50 mole% or more of this side comprises an amino acid selected from the group consisting of arginine and lysine,
   the other of said hydrophobic sides comprises 2 to 4 hydrophobic amino acids, and
   the other of said hydrophilic sides comprises 3 to 5 amino acids and 80 mole% or more of this side comprises hydrophilic amino acids.
(2) A peptide chemically modified with PEG according to (1) wherein said peptide comprises 20 or more amino acids in total; opposite ends of said peptide are N and C terminals; and any 18 consecutive amino acids in said peptide excluding the amino acids at opposite ends constitutes said sequence of 18 amino acids.
(3) A peptide chemically modified with PEG according to (1) or (2) wherein the amino acids at the N and C terminals are each a hydrophilic amino acid.
(4) A peptide chemically modified with PEG according to any one of (1) to (3) wherein said sequence of 18 amino acids is a sequence of any 18 consecutive amino acids in the following amino acid sequence:
   X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36,
   provided that,
   in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
   X3, X10, X12, X21, X28, and X30 are independently a hydrophobic amino acid, a neutral hydrophilic amino acid, or a basic hydrophilic amino acid,
   in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
   X6, X17, X24, and X35 are independently a hydrophobic amino acid, and
   X7, X14, X18, X25, X32, and X36 are independently a neutral hydrophilic amino acid or a basic hydrophilic amino acid.
(5) A peptide chemically modified with PEG according to any one of (1) to (4) wherein peptide moiety of said peptide chemically modified with PEG and including said sequence of 18 amino acids comprises the following amino acid sequence:
   X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36-X37,
   provided that
   X1 and X37 are a hydrophilic amino acid,
   in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
   X3, X10, X12, X21, X28 and X30 are independently a hydrophobic amino acid, a neutral hydrophilic amino acid, or a basic hydrophilic amino acid,
   in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
   X6, X17, X24, and X35 are independently a hydrophobic amino acid, and
   X7, X14, X18, X25, X32, and X36 are independently a neutral hydrophilic amino acid or a basic hydrophilic amino acid; and
   wherein the sequence of amino acids X2 to X36 may include deletion, addition, insertion, or substitution as long as at least 18 amino acids are conserved in consecutive form.
(6) A peptide chemically modified with PEG according to (5) wherein X1 to X37 are the following amino acids:
   X1 is threonine,
   X37 is serine,
   X2, X5, X9, X20, X23, and X27 are independently arginine or lysine,
   X3 and X21 are independently tyrosine, phenylalanine, serine, or arginine,
   X4, X17, X22, and X35 are independently leucine,
   X6, X15, X24, and X33 are independently leucine or isoleucine,
   X7, X13, X25, and X31 are independently histidine or arginine,
   X8 and X26 are independently proline,
   X10 and X28 are independently serine, arginine, or leucine,
   X11 and X29 are independently tryptophan or leucine,
   X12 and X30 are independently valine, leucine, or serine,
   X14 and X32 are independently glutamine, asparagine, or arginine,
   X16 and X34 are independently alanine or arginine,
   X18 is arginine, lysine, or serine,
   X19 is leucine or threonine, and
   X36 is arginine or serine; and
   wherein the sequence of amino acids X2 to X36 may include deletion, addition, insertion, or substitution as long as at least 18 amino acids are conserved in consecutive form.
(7) A peptide chemically modified with PEG according to any one of (1) to (6) wherein peptide moiety of said peptide chemically modified with PEG and including said sequence of 18 amino acids comprises any one of the amino acid sequences of SEQ ID NO: 1 to SEQ ID NO: 24.
(8) A peptide chemically modified with PEG according to any one of (1) to (6) wherein peptide moiety of said peptide chemically modified with PEG and including said sequence of 18 amino acids comprises the amino acid sequence of SEQ ID NO: 16 or SEQ ID NO: 19.
(9) A peptide chemically modified with PEG according to any one of (1) to (8) wherein PEG moiety of said peptide chemically modified with PEG and including said sequence of 18 amino acids has a molecular weight of about 200 Da to about 100,000 Da.
   The second aspect of the present invention is as follows:
(10) A complex comprising a peptide chemically modified with PEG and including a sequence of 18 amino acids, and a substance which binds to said peptide wherein
   said sequence of 18 amino acids is constituted from alternately arranged two hydrophobic sides and two hydrophilic sides in α-helix structural model depicted by Edmundson wheel plots,
   one of said hydrophobic sides comprises 5 to 7 amino acids and 80 mole% or more of this side comprises hydrophobic amino acids,
   one of said hydrophilic sides comprises 5 or 6 amino acids, and 80 mole% or more of this side comprises hydrophilic amino acids, and 50 mole% or more of this side comprises an amino acid selected from the group consisting of arginine and lysine,
   the other of said hydrophobic sides comprises 2 to 4 hydrophobic amino acids, and
   the other of said hydrophilic sides comprises 3 to 5 amino acids and 80 mole% or more of this side comprises hydrophilic amino acids.
(11) A complex according to (10) wherein said peptide comprises 20 or more amino acids in total; opposite ends of said peptide are N and C terminals; and any 18 consecutive amino acids in said peptide excluding the amino acids at opposite ends constitutes said sequence of 18 amino acids.
(12) A complex according to (10) or (11) wherein the amino acids at the N and C terminals are each a hydrophilic amino acid.
(13) A complex according to any one of (10) to (12) wherein said sequence of 18 amino acids is a sequence of any 18 consecutive amino acids in the following amino acid sequence:
   X2-X3-X4-X5-X6-X7-X8-X9-X10-X11-X12-X13-X14-X35-X16-X17-X18-X19-X20-X21-X22-X23-X24-X25-X26-X27-X28-X29-X30-X31-X32-X33-X34-X35-X36,
   provided that,
   in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
   X3, X10, X12, X21, X28, and X30 are independently a hydrophobic amino acid, a neutral hydrophilic amino acid, or a basic hydrophilic amino acid,
   in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
   X6, X17, X24, and X35 are independently a hydrophobic amino acid, and
   X7, X14, X18, X25, X32, and X36 are independently a neutral hydrophilic amino acid or a basic hydrophilic amino acid.
(14) A complex according to any one of (10) to (13) wherein peptide moiety of said peptide chemically modified with PEG and including said sequence of 18 amino acids comprises the following amino acid sequence:
   X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36-X37,
   provided that
   X1 and X37 are a hydrophilic amino acid,
   in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
   X3, X10, X12, X21, X28 and X30 are independently a hydrophobic amino acid, a neutral hydrophilic amino acid, or a basic hydrophilic amino acid,
   in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
   X6, X17, X24, and X35 are independently a hydrophobic amino acid, and
   X7, X14, X18, X25, X32, and X36 are independently a neutral hydrophilic amino acid or a basic hydrophilic amino acid; and
   wherein the sequence of amino acids X2 to X36 may include deletion, addition, insertion, or substitution as long as at least 18 amino acids are conserved in consecutive form.
(15) A complex according to (14) wherein X1 to X37 are the following amino acids:
   X1 is threonine,
   X37 is serine,
   X2, X5, X9, X20, X23, and X27 are independently arginine or lysine,
   X3 and X21 are independently tyrosine, phenylalanine, serine, or arginine,
   X4, X17, X22, and X35 are independently leucine,
   X6, X15, X24, and X33 are independently leucine or isoleucine,
   X7, X13, X25, and X31 are independently histidine or arginine,
   X8 and X26 are independently proline,
   X10 and X28 are independently serine, arginine, or leucine,
   X11 and X29 are independently tryptophan or leucine,
   X12 and X30 are independently valine, leucine, or serine,
   X14 and X32 are independently glutamine, asparagine, or arginine,
   X16 and X34 are independently alanine or arginine,
   X18 is arginine, lysine, or serine,
   X19 is leucine or threonine, and
   X36 is arginine or serine; and
   wherein the sequence of amino acids X2 to X36 may include deletion, addition, insertion, or substitution as long as at least 18 amino acids are conserved in consecutive form.
(16) A complex according to any one of (10) to (15) wherein peptide moiety of said peptide chemically modified with PEG and including said sequence of 18 amino acids comprises any one of the amino acid sequences of SEQ ID NO: 1 to SEQ ID NO: 24.
(17) A complex according to any one of (10) to (15) wherein peptide moiety of said peptide chemically modified with PEG and including said sequence of 18 amino acids comprises the amino acid sequence of SEQ ID NO: 16 or SEQ ID NO: 19.
(18) A complex according to any one of (10) to (17) wherein said substance which binds to the peptide is a nucleic acid.
(19) A complex according to any one of (10) to (18) wherein PEG moiety of said peptide chemically modified with PEG and including said sequence of 18 amino acids has a molecular weight of about 200 Da to about 100,000 Da.
(20) A method for producing the peptide chemically modified with PEG of any one of (1) to (9) comprising the step of reacting a peptide comprising said sequence of 18 amino acids with activated polyethylene glycol.
(21) A peptide chemically modified with polyethylene glycol (PEG) which is produced by the method of (20).
(22) A method for producing the complex of any one of (10) to (19) comprising the steps of
   a) reacting a peptide comprising said sequence of 18 amino acids with activated polyethylene glycol (PEG), and
   b) reacting the peptide chemically modified with PEG that is obtained in said a) with a substance which binds to said peptide.
(23) A method for producing the complex of any one of (10) to (19) comprising the steps of
   a) reacting a peptide comprising said sequence of 18 amino acids with a substance which binds to said peptide, and
   b) reacting the reaction product of said peptide and said substance which binds to said peptide with activated polyethylene glycol (PEG).
(24) A complex of a peptide chemically modified with polyethylene glycol (PEG) and a substance which binds to said peptide, said complex being produced by the method of (22) or (23).
(25) A carrier which is modified with the peptide chemically modified with PEG according to any one of (1) to (8).
(26) A method for producing the carrier of (25) which is modified with the peptide chemically modified with PEG comprising the steps of
   a) reacting a peptide comprising said sequence of 18 amino acids, or a peptide comprising said sequence of 18 amino acids and having cysteine attached to N or C terminal of the peptide, with activated PEG, and
   b) reacting the reaction product of said a) with a carrier, or constructing a carrier by using the reaction product of said a) as a constituent.
(27) A carrier which is modified with the peptide chemically modified with PEG, said carrier being produced by the method of (26).
(28) A method for delivering a substance to the interior of a cell, said substance being bonded to or incorporated in the carrier of (25) that has been modified with the peptide chemically modified with PEG.

### BRIEF DESCRIPTION OF THE DRAWINGS

FIGs. 1(A), 1(B), and 1(C) are schematic views showing α-helix structural model by Edmundson wheel plots. (In the figures, the amino acids surrounded by square are hydrophobic amino acids, the amino acids surrounded by circle are basic hydrophilic amino acids, and the amino acids not surrounded by any of these are neutral hydrophilic amino acids. The same also applies to other figures).
FIG. 2 is a view showing an exemplary four sided structure of the sequence of 18 amino acids in the peptide used in the present invention.
FIGs. 3(A) and 3(B) are views showing an exemplary four sided structure of the peptide used in the present invention with the amino acids allocated to respective sides in a different manner.
FIG. 4(a) shows (C1), and FIG. 4(b) shows (C2). (C1) and (C2) are views showing an exemplary four sided structure of the peptide used in the present invention with the amino acids allocated to respective sides in a different manner.
FIG. 5(A) and FIG. 5(B) are views showing an exemplary four sided structure of the peptide of the present invention including the sequence of 18 amino acids.
FIG. 6(a) shows (C), and FIG. 6(b) shows (D). (C) and (D) are views showing an exemplary four sided structure of the peptide of the present invention including the sequence of 18 amino acids.
FIG. 7(a) is a view showing mean residue ellipticity in CD spectroscopy under SDS(-) conditions. FIG. 7(b) is a view showing mean residue ellipticity in CD spectroscopy under SDS(+) conditions.
FIG. 8(a) is a view showing mean residue ellipticity in CD spectroscopy under SDS(-) conditions. FIG. 8(b) is a view showing mean residue ellipticity in CD spectroscopy under SDS(+) conditions.
FIG. 9(a) is a view showing mean residue ellipticity in CD spectroscopy under SDS(-) conditions. FIG. 9(b) is a view showing mean residue ellipticity in CD spectroscopy under SDS(+) conditions.
FIG. 10 is an electropherogram of the mixture of the peptide of SEQ ID NO: 1 and an oligonucleotide.
FIG. 11 is an electropherogram of the mixture of the peptide of SEQ ID NO: 1 and a plasmid.
FIG. 12 is a view showing how a plasmid was introduced in the cell in the presence and absence of the peptide of SEQ ID NO: 1 by using the luciferase activity expressed by the plasmid as the index.
FIG. 13 is a view showing increase of GCV sensitivity when a plasmid including HSV-tk gene was introduced in a cell by using the peptide of SEQ ID NO: 16.
FIG. 14 is a view showing the ability of the peptide of SEQ ID NO: 1 for introducing a plasmid in a cell before and after storing the complex of the peptide with the plasmid at 4°C by using the luciferase activity expressed by the plasmid as the index.
FIG. 15 is an electropherogram of an oligonucleotide after treating the mixture of the peptide of SEQ ID NO: 1 and the oligonucleotide with a nuclease.
FIG. 16 is an electropherogram of a plasmid after treating the mixture of the peptide of SEQ ID NO: 16 and the plasmid with a nuclease. Control is the plasmid which has not been treated with the nuclease.
FIG. 17 is a view showing the specific affinity of the peptide of SEQ ID NO: 1 for phosphatidyl serine which has been measured by using Biacore 2000.
FIG. 18 is a view showing the measurements obtained by using Biacore 2000. The measurements indicate that the peptide of SEQ ID NO: 25 has no affinity for either phosphatidyl serine or phosphatidyl choline.
FIG. 19 is a view showing the results of flow cytometry showing that, when a cell is stimulated for degranulation, phosphatidyl serine is translocated to the surface of the cell.
FIG. 20 is a view showing that the peptide of SEQ ID NO: 16 introduces a larger amount of gene into the cell which has undergone degranulation with the phosphatidyl serine translocated to its surface.
FIG. 21 is a view showing that the peptide of SEQ ID NO: 16 is capable of introducing a plasmid into the cancer cell that had been transplanted in a mouse, by using the luciferase activity expressed by the plasmid as the index.
FIG. 22 is a view showing that survival period of a mouse can be extended by introducing the plasmid containing HSV-tk gene in the cancer cell that had been transplanted in a mouse by using the peptide of SEQ ID NO: 16, and thereafter administering GCV.
FIG. 23 is a graph wherein gene introduction ability is compared between the PEG-modified plasmid / peptide complex and the corresponding PEG-unmodified complex by using Vero cell.
FIG. 24 is a graph wherein gene introduction ability is compared between the PEG-modified plasmid / peptide complex and the corresponding PEG-unmodified complex by using anaphylactic shock mice.
FIG. 25 shows the results of SDS-PAGE wherein PEG-modified peptide in the PEG-modified plasmid / peptide complex was identified.

### BEST MODE FOR CARRYING OUT THE INVENTION

Next, the present invention is described in further detail.

The peptide chemically modified with PEG of the present invention (according to the first aspect of the present invention) is a peptide chemically modified with PEG (hereinafter referred to as "the peptide chemically modified with PEG") including an amino acid sequence comprising 18 amino acids, wherein said sequence of 18 amino acids is constituted from four sides comprising alternately arranged two hydrophobic sides (side A and side C) and two hydrophilic sides (side B and side D) in α-helix structural model depicted by Edmundson wheel plots (Edmundson, A.B. et al., Biophys. J., 7, 121 (1967)), and at least one side (side B) of the two hydrophilic sides (side B and side D) is a positively charged side.

The peptide chemically modified with PEG according to the present invention is obtained by chemically modifying the peptide containing the sequence comprising 18 amino acids (hereinafter abbreviated as "the peptide used in the present invention") as will be described below, for example, with activated PEG.

The method used for the chemical modification with PEG is not limited to any particular method, and exemplary methods include those using an activated PEG and those using PEG and an activating agent.

The activated PEG used for the chemical modification is not particularly limited as long as it is an activated PEG, and may comprise a straight chain or a branched chain structure. Examples of the activated PEG include mPEG-SPA (succinimidyl ester of methoxy poly(ethylene glycol) propionic acid manufactured by Shearwater) and other products of Shearwater such as mPEG-SBA (succinimidyl ester of mPEG butanoic acid), mPEG-SS (succinimidyl ester of mPEG succinate), mPEG-SCM (succinimidyl ester of carboxymethylated mPEG), mPEG-BTC (benzotriazole carbonate derivative of mPEG), mPEG-epoxide, mPEG-CDI (carbonyldiimidazole-activated mPEG), mPEG-NPC (p-nitrophenyl mPEG carbonate), mPEG-aldehyde, mPEG-Isocyanate, mPEG-maleimide, and mPEG-OPSS (mPEG orthopyridyl disulfide). A PEG synthesized by a known method is also useful.

The activated PEG used for the chemical modification may also be PEG having a phospholipid attached thereto, for example, MAL-mPEG-DSPE (maleimide modified and distearoylphosphatidylethanolamine modified mPEG), and NHS-mPEG-DSPE (N-hydroxysuccinimidyl carbonate modified mPEG-DSPE).

The average molecular weight of PEG may range from about 200 Da to about 100,000 Da, and preferably about 1,000 Da to about 50,000 Da, and more preferably about 2,000 Da to about 20,000 Da.

When the average molecular weight is within such range, the PEG-modified peptide will retain the activity of the original peptide after the reaction between the activated PEG and the peptide, and the PEG-modified peptide will be allowed to have favorable characters such as improved solubility, reduced antigenicity, and reduced toxicity.

The PEG and the activating agent used in the chemical modification with the PEG and the activating agent, and the like may be selected from those commonly used in the art.

The site of modification (bonding) with the PEG in the peptide chemically modified with PEG of the present invention is not particularly limited. The site of modification, however, is preferably a site other than the sites of the bonding of the peptide used in the present invention with the peptide-binding substance such as nucleic acid, and phosphatidyl serine or the like, and more preferably, the site of modification is at N terminal, C terminal, or on the side chain.

The method used for introducing the PEG into the desired site in the peptide may be any method known in the art.

The amount of PEG for the modification of (namely, PEG to be bonded to) the peptide used in the present invention is not limited to any particular amount.

By modifying the peptide used in the present invention with the activated PEG, improvements of the characters and properties (which are described below) inherent to the original peptide have been enabled.

Such improvements enabled for the peptide used in the present invention include improvement in the rate of incorporation of the genes and the drugs into the target cell, improvement in the pharmacological activity, decrease in the toxicity, and other excellent effects.

Next, "the amino acid sequence comprising 18 amino acids", which is a structure critical in the peptide used in the present invention that is a peptide including a sequence of 18 amino acids, is described by referring to the drawings.

Edmundson wheel plot is a model which shows position of the amino acids in relation to the central axis of the α-helix, and this plot is depicted so that the wheel is completed by 18 amino acids and the 19th amino acid comes to the same position as the 1st amino acid.

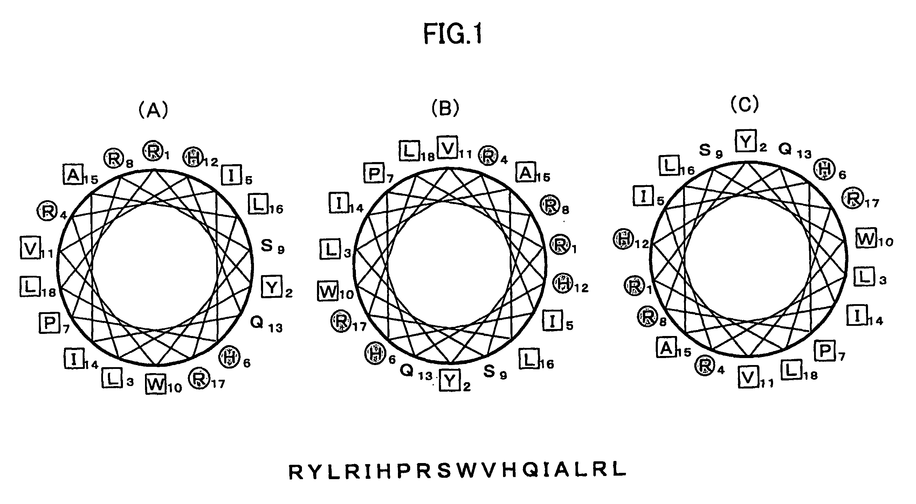

In the model depicted by this method, the first amino acid located at the starting point is typically depicted at the position of 12 o'clock in a clock. There is, however, no difference in the relative location of the amino acids in the plot if the plotting were started from a different position in the drawing as long as the amino acid sequence is the same. For example, (A), (B), and (C) are essentially identical in FIG. 1.

In the present invention, the "side" designates an area of the model constituted by consecutive and adjacent amino acids. The number of amino acids constituting each side may be one or more, and preferably, each side may comprise two or more amino acids.

"Consecutive and adjacent" designates, for example, the positional relation how the 1st and the 12th amino acids, or four amino acids, namely, the 15th, the 8th, the 1st, and the 12th amino acids are located in FIG. 1. In contrast, the 1st and the 10th amino acids are not located in consecutive manner. The 1st and the 5th amino acids are also not located in "consecutive and adjacent" manner, while the 1st and 5th amino acids may constitute the same side together with the adjoining 12th amino acid.

The "hydrophobic side" is a side which includes a substantial number of hydrophobic amino acids. The hydrophobic amino acid is not particularly limited as long as it is substantially hydrophobic, and the hydrophobic amino acid may be a natural hydrophobic amino acid, or a modified or synthetic amino acid having characteristic features nearly equivalent to those of the natural amino acid.

The peptide used in the present invention may preferably include 80 mole% or more of hydrophobic amino acids in the hydrophobic side, and more preferably, no acidic hydrophilic amino acid (aspartic acid and glutamic acid) in the hydrophobic side since the acidic hydrophilic amino acid interferes with the electrostatic binding formed between the positively charged moiety of the peptide and the negatively charged moiety of the nucleic acid.

The hydrophobic amino acids are preferably those selected from leucine, isoleucine, valine, tryptophan, proline, tyrosine, alanine, phenylalanine, methionine, cysteine, and glycine. One side (side A) of the hydrophobic sides may preferably comprise 5 to 7 amino acids. The other hydrophobic side (side C) may preferably comprise 2 to 4 amino acids. It is particularly preferable that side C comprises solely from hydrophobic amino acids.

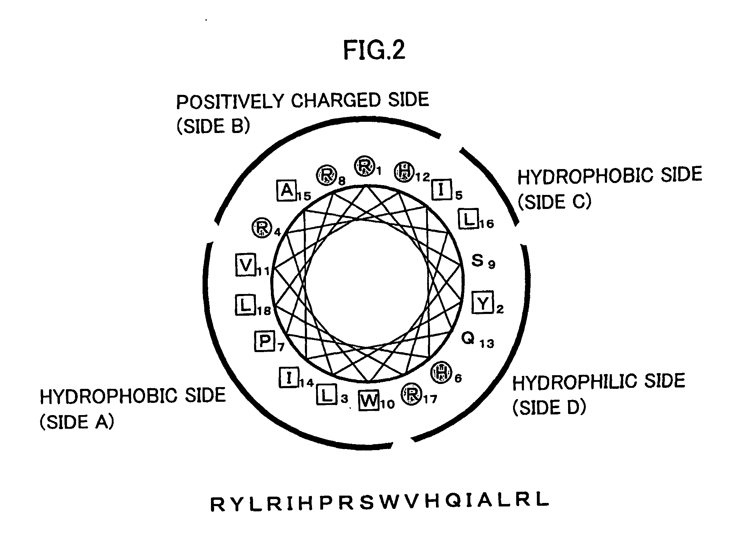

Typical hydrophobic sides (side A and side C) are shown in FIG. 2.

The "hydrophilic side" is a side which includes a substantial number of hydrophilic amino acids. The hydrophilic amino acid is not particularly limited as long as it is substantially hydrophilic, and the hydrophilic amino acid may be a natural hydrophilic amino acid, or a modified or synthetic amino acid having characteristic features nearly equivalent to those of the natural amino acid.

The "positively charged side" is a "hydrophilic side" which includes a considerable number of substantially positively charged hydrophilic amino acids. The hydrophilic amino acid may be a substantially positively charged, natural hydrophilic amino acid, or a modified or synthetic amino acid having characteristic features nearly equivalent to those of the natural amino acid.

The peptide used in the present invention may preferably contain 80 mole% or more of hydrophilic amino acids in the hydrophilic side, and the hydrophilic amino acids are preferably those selected from asparagine, glutamine, threonine, serine, arginine, histidine, lysine, aspartic acid, and glutamic acid. It is more preferable that the hydrophilic amino acids are those other than acidic hydrophilic amino acids, namely, those selected from asparagine, glutamine, threonine, serine, arginine, histidine, and lysine since the acidic hydrophilic amino acid interferes with the electrostatic binding formed between the positively charged moiety of the peptide and the negatively charged moiety of the nucleic acid.

One side (side B) of the hydrophilic sides is preferably a positively charged side comprising 5 to 6 amino acids. More preferably, 50 mole% or more of the amino acids constituting this side are selected from lysine and arginine.

The other hydrophilic side (side D) may preferably comprise 3 to 5 amino acids.

Typical hydrophilic side (side D) and positively charged side (side B) are shown in FIG. 2.

It is to be noted that the "mole%" used herein designates the ratio of the number of hydrophobic amino acids in the hydrophobic side to the number of amino acids constituting the hydrophobic side, the ratio of the number of hydrophilic amino acids in the hydrophilic side to the number of amino acids constituting the hydrophilic side, or the ratio of the number of amino acids selected from lysine and arginine in the positively charged side to the number of amino acids constituting the positively charged side.

The four sided structure comprising the alternately arranged two hydrophobic sides and two hydrophilic sides (wherein at least one of the hydrophilic sides is a positively charged side) which is the characteristic feature of the peptide used in the present invention is a structure wherein, when the 18 amino acids shown in the model is divided into the sides in accordance with the definition as described above, two hydrophobic sides (side A and side C) and two hydrophilic sides (side B and side D, wherein at least side B is a positively charged side) are alternately arranged to constitute the four side.

It is to be noted that, when a hydrophobic side is directly juxtaposed to another hydrophobic side, these sides are not regarded as two hydrophobic sides but as one integrated hydrophobic side; and when a hydrophilic side is directly juxtaposed to another hydrophilic side, these sides are regarded not as two hydrophilic sides but one integral hydrophilic side; and when a positively charged side is directly juxtaposed to another positively charged side, these sides are regarded not as two positively charged sides but as one integral positively charged side.

In other words, in the four sided structure, the hydrophobic side is not adjacent to another hydrophobic side, the hydrophilic side is not adjacent to another hydrophilic side, and the positively charged side is not adjacent to another positively charged side.

"The structure wherein two hydrophobic sides and two hydrophilic sides are alternately arranged to constitute the four side" is not particularly limited as long as the four sides are arranged [side A → side B → side C → side D (→ side A)] in clockwise or [side A → side D → side C → side B (→ side A)] in clockwise in the α-helix structural model of Edmundson wheel plot, and the function is retained. The preferable sequence is the one wherein sides are arranged [side A → side B → side C → side D (→ side A)] in clockwise. It is to be noted that the four sided structure is not limited for its method how the amino acids are divided, namely, how the 18 amino acids are allocated to each of four sides as long as each side retains its character.

The amino acids are preferably allocated on the basis of the amino acid sequence such that side A comprises 5 to 7 amino acids, side B comprises 5 to 6 amino acids, and side C comprises 2 to 4 amino acids, and side D comprises 3 to 5 amino acids.

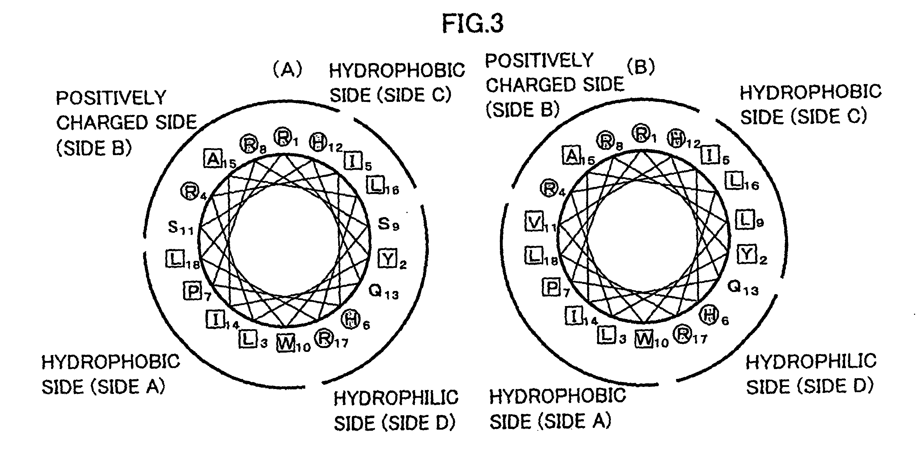

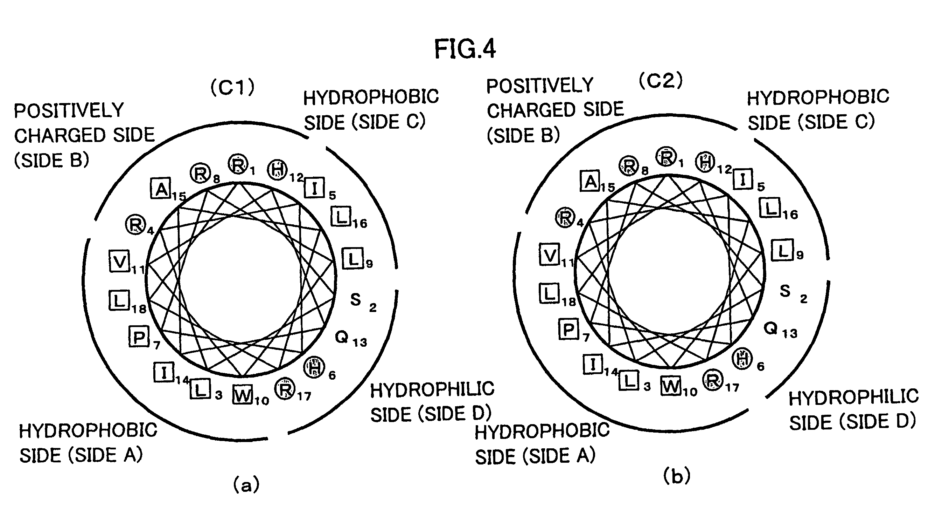

Typical three sequences wherein the amino acid sequence is allocated to each side in a different manner are shown as sequence A to C (C1 and C2) in FIGS. 3 and 4. It is to be noted that the amino acid sequence of C1 and C2 is completely the same, and the different allocations are both within the scope of the definition as described above. The allocation of amino acids in A to C (C1 and C2) are as described below.

| | Hydrophobic side | Hydrophilic side | Hydrophobic side | Hydrophilic side |
|---|---|---|---|---|
| | (Side A) | (Side B) | (Side C) | (Side D) |
| A | 5 | 6 | 2 | 5 |
| B | 6 | 5 | 4 | 3 |
| C1 | 6 | 5 | 3 | 4 |
| C2 | 7 | 5 | 3 | 3 |

Since the peptide used in the present invention contains the amino acid sequence comprising 18 amino acids exhibiting the four sided structure as its characteristic feature as described above, the peptide has excellent solubility in water. The peptide also has a characteristic feature that it is capable of forming a complex with a peptide-binding substance without forming aggregates of the complex which may cause a substantial problem.

In contrast to the peptide vector having an α-helix structure which has been described in the section of "Prior Art", in the case of the peptide used in the present invention, a plurality of hydrophobic sides are formed when the peptide takes α-helix structure, and therefore, the peptide has high ability of introducing a peptide-binding substance into the cell even when the proportion of the hydrophobic sides is reduced for the purpose of improving the solubility in water. A plurality of hydrophilic sides are also formed simultaneously, and accordingly, proportion of the hydrophilic sides is higher compared to the peptide of two sided structure, and the hydrophilicity of the peptide does not completely disappear even when the peptide-binding substance becomes electrostatically bonded to the at least one positively charged side of the peptide. Solubility in water of the complex of the peptide and the peptide-binding substance is thereby maintained, and as a consequence, formation of troublesome aggregate masses is prevented. As described above, if the peptide were to have a high peptide-binding substance-introducing ability simultaneously with an excellent solubility in water, a plurality of hydrophobic sides and a plurality of hydrophilic sides (of which at least one side is the positively charged side) need to be formed when the peptide takes α-helix structure.

The peptide chemically modified with PEG of the present invention, as including the peptide of above characters used in the present invention, is further improved in such characters and properties.

It should also be noted that the peptide is not limited for its number of sides as long as two or more hydrophobic sides and two or more hydrophilic sides are formed, and the function as a peptide vector is maintained. However, the peptide may preferably have two hydrophobic and two hydrophilic sides, and in particular, at least one side of the two or more hydrophilic sides is preferably a side rich in neutral hydrophilic amino acids (namely, a side with no substantial charge), since such side will not become bonded to the peptide-binding substance and water solubility of such side will substantially be fully maintained.

The peptide used in the present invention is by no means limited for its length of the amino acid sequence as long as its function is retained. The peptide, however, is preferably the one having a total amino acid residue number of 20 or more, more preferably 25 or more, and most preferably 30 or more. The peptide may preferably have a total amino acid residue number of up to 100, more preferably up to 50, and most preferably up to 40.

The peptide used in the present invention includes at least one amino acid sequence of 18 consecutive amino acids starting from any amino acid. Preferably, the peptide of the present invention is a peptide containing at least two independent amino acid sequences of 18 consecutive amino acids starting from any amino acid; and/or at least two overlapping amino acid sequences of 18 consecutive amino acids. More preferably, the peptide of the present invention is a peptide wherein any consecutive 18 amino acids excluding the amino acids at the opposite ends represents the amino acid sequence of 18 amino acids exhibiting the four sided structure of the present invention, that is, a peptide wherein all of the overlapping amino acid sequences comprising 18 consecutive amino acids excluding the amino acids at the opposite ends exhibits the four sided structure of the present invention.

"The amino acid sequence comprising 18 amino acids" used herein designates an amino acid sequence comprising 18 amino acids wherein side A, side B, side C, and side D are arranged in clockwise direction in the α-helix model by Edmundson wheel plot (hereinafter referred to as "the four sided structure of the present invention") (Such sequence is hereinafter referred to as "the amino acid sequence comprising 18 amino acids exhibiting the four sided structure of the present invention").

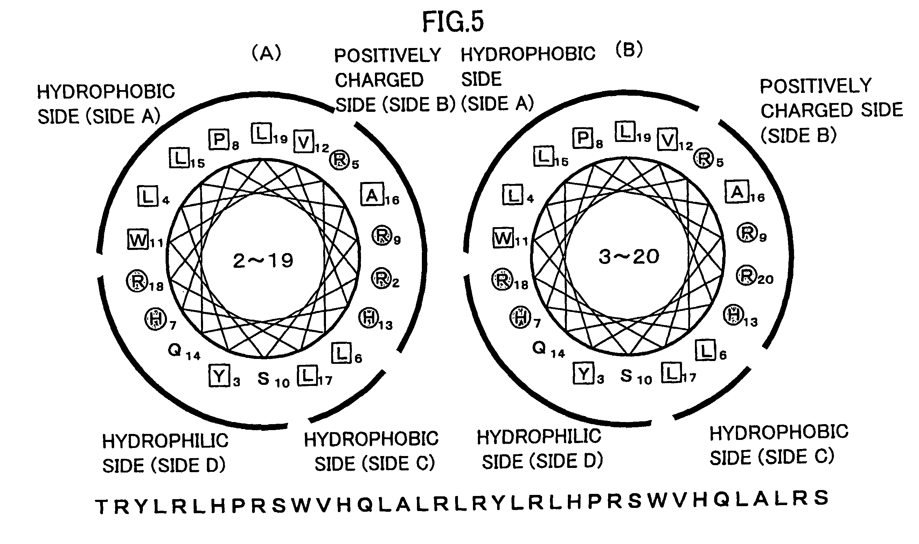

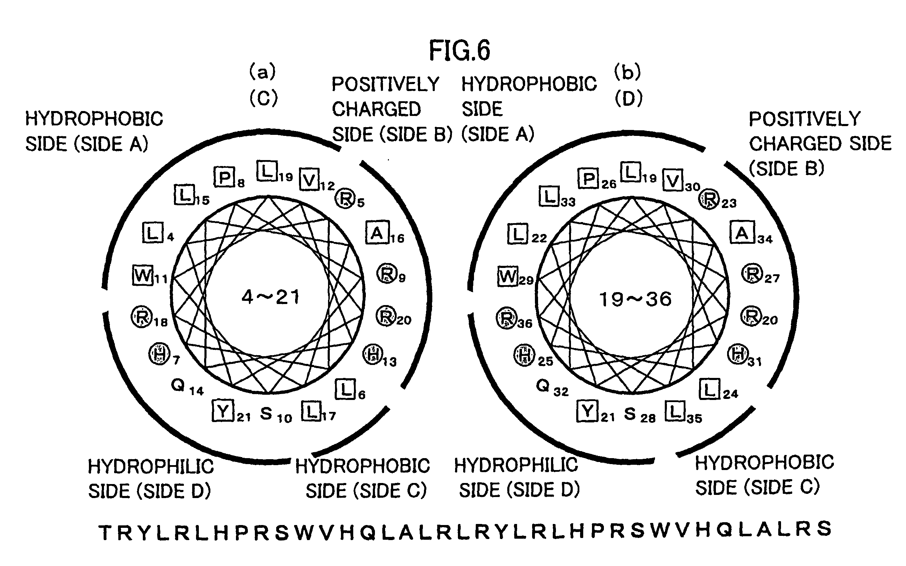

"The amino acid sequence comprising any consecutive 18 amino acids excluding the amino acids at opposite ends" means, for example, any amino acid sequence in a peptide comprising N amino acids (wherein N represents a number of 20 or more) comprising 2nd to 19th, 3rd to 20th, 4th to 21st, or in a similar manner, (N-18)th to (N-1)th amino acids. "All of the overlapping amino acid sequences comprising 18 consecutive amino acids excluding the amino acids at the opposite ends exhibits the four sided structure of the present invention" means that all of the above-mentioned sequences comprising 2nd to 19th, 3rd to 20th, 4th to 21st, or in a similar manner, (N-18)th to (N-1)th amino acids exhibit the four sided structure of the present invention. Examples of such amino acid sequence are shown in FIGS. 5 and 6 as sequences A to D in the α-helix model by Edmundson wheel plot, and the sequences shown are the sequences comprising 18 amino acids of from 2nd to 19th, from 3rd to 20th, from 4th to 21st, and from 19 to 36th amino acids in the peptide of SEQ ID NO: 16. All of these sequences show the four sided structure of the present invention.

In view of further improving the solubility of the complex of the peptide and the substance which binds to the peptide, the peptide used in the present invention is preferably the one wherein at least one end comprises a hydrophilic amino acid, and more preferably, the one wherein both ends comprise a hydrophilic amino acids.

Exemplary such peptide-binding substances include nucleic acids, acidic high molecular weight compounds such as acidic protein, and physiologically active low molecular weight compounds having a negatively charged side chain (as will be further described below).

The hydrophilic amino acid is not particularly limited as long as it is hydrophilic. The hydrophilic amino acid, however, is preferably the one other than acidic hydrophilic amino acid, and more preferably, a neutral hydrophilic amino acid, and most preferably threonine or serine.

Preferable examples of "the amino acid sequence of 18 amino acids exhibiting the four sided structure of the present invention" included in the peptide used in the present invention are the amino acid sequences comprising any consecutive 18 amino acids in the following amino acid sequence:
X2-X3-X4-XS-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36,
provided that,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
X3, X10, X12, X21, X28, and X30 are independently a member selected from a hydrophobic amino acid, a neutral hydrophilic amino acid and a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
X6, X17, X24, and X35 are independently a hydrophobic amino acid, and

X7, X14, X18, X25, X32, and X36 are independently a neutral hydrophilic amino acid or a basic hydrophilic amino acid.
Preferably,
X8 and X26 are proline,
X4, X17, X22, and X35 are leucine,
X6, X11, X15, X24, X29, and X33 are independently a hydrophobic amino acid,
X12, X19, and X30 are independently a hydrophobic amino acid or a neutral hydrophilic amino acid,
X2, X5, X9, X20, X23, and X27 are independently a basic hydrophilic amino acid,
X13 and X31 are independently a basic hydrophilic amino acid or a neutral hydrophilic amino acid,
X16 and X34 are independently a hydrophobic amino acid or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 3 amino acid in the 5 amino acids are arginine or lysine,
X3, X10, X21, and X28 are independently a member selected from a hydrophobic amino acid, a neutral hydrophilic amino acid, and a basic hydrophilic amino acid, and
X7, X14, X18, X25, X32, and X36 are independently a neutral hydrophilic amino acid or a basic hydrophilic amino acid.
More preferably,
X2, X5, X9, X20, X23, and X27 are independently arginine or lysine,
X3 and X21 are independently a member selected from tyrosine, phenylalanine, serine, and arginine,
X4, X17, X22, and X35 are independently leucine,
X6, X15, X24, and X33 are independently leucine or isoleucine,
X7, X13, X25, and X31 are independently histidine or arginine,
X8 and X26 are independently proline,
X10 and X28 are independently a member selected from serine, arginine, and leucine,
X11 and X29 are independently tryptophan or leucine,
X12 and X30 are independently valine, leucine, or serine,
X14 and X32 are independently a member selected from glutamine, asparagine, and arginine,
X16 and X34 are independently alanine or arginine,
X18 is a member selected from arginine, lysine and serine,
X19 is leucine or threonine, and
X36 is arginine or serine.

In this connection, the sequence of amino acids X2 to X36 may include deletion, addition, insertion, or substitution as long as at least 18 amino acids are conserved in consecutive form.

It should be noted that, in the above description, a hydrophobic amino acid is an amino acid selected from leucine, isoleucine, valine, tryptophan, proline, tyrosine, alanine, cysteine, phenylalanine, methionine, and glycine; a basic hydrophilic amino acid is an amino acid selected from arginine, histidine, and lysine; and a neutral hydrophilic amino acid is an amino acid selected from asparagine, glutamine, threonine, and serine.

A preferable example of the peptide used in the present invention is a peptide comprising the following amino acid sequence:
X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36-X37,
provided that
X1 and X37 are a hydrophilic amino acid,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
X3, X10, X12, X21, X28 and X30 are independently a member selected from a hydrophobic amino acid, a neutral hydrophilic amino acid, and basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
X6, X17, X24, and X35 are independently a hydrophobic amino acid, and

X7, X14, X18, X25, X32, and X36 are independently a neutral hydrophilic amino acid or a basic hydrophilic amino acid.

It is to be noted that in the amino acid sequence as described above, the sequence of amino acids X2 to X36 may include deletion, addition, insertion, or substitution as long as at least 18 amino acids are conserved in consecutive form.
Preferably,
X1 and X37 are independently threonine or serine,
X8 and X26 are independently proline,
X4, X17, X22, and X35 are independently leucine,
X6, X11, X15, X24, X29, and X33 are independently a hydrophobic amino acid,
X12, X19, and X30 are independently a hydrophobic amino acid or a neutral hydrophilic amino acid,
X2, X5, X9, X20, X23, and X27 are independently a basic hydrophilic amino acid,
X13 and X31 are independently a basic hydrophilic amino acid or a neutral hydrophilic amino acid,
X16 and X34 are independently a hydrophobic amino acid or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 3 amino acids in the 5 amino acids is arginine or lysine,
X3, X10, X21, and X28 are independently a member selected from a hydrophobic amino acid, a neutral hydrophilic amino acid, and a basic hydrophilic amino acid, and
X7, X14, X18, X25, X32, and X36 are independently a neutral hydrophilic amino acid or a basic hydrophilic amino acid.
It is to be noted that the sequence of amino acids X2 to X36 may include deletion, addition, insertion, or substitution as long as at least 18 amino acids are conserved in consecutive form.
More preferably,
X1 is threonine,
X37 is serine,
X2, X5, X9, X20, X23, and X27 are independently arginine or lysine,
X3 and X21 are independently a member selected from tyrosine, phenylalanine, serine and arginine,
X4, X17, X22, and X35 are independently leucine,
X6, X15, X24, and X33 are independently leucine or isoleucine,
X7, X13, X25, and X31 are independently histidine or arginine,
X8 and X26 are independently proline,
X10 and X28 are independently a member selected from serine, arginine, and leucine,
X11 and X29 are independently tryptophan or leucine,
X12 and X30 are independently a member selected from valine, leucine and serine,
X14 and X32 are independently a member selected from glutamine, asparagine and arginine,
X16 and X34 are independently alanine or arginine,
X18 is a member selected from arginine, lysine and serine,
X19 is leucine or threonine, and
X36 is arginine or serine.

It is to be noted that in the amino acid sequence as described above, the sequence of amino acids X2 to X36 may include deletion, addition, insertion, or substitution as long as at least 18 amino acids are conserved in consecutive form.

In the above description, the hydrophobic amino acid, the basic hydrophilic amino acid, and the neutral hydrophilic amino acid are the same as those described before for the amino acid sequence comprising amino acids X2 to X36.

It is to be noted that the amino acid sequences as mentioned above are only some examples of the peptide used in the present invention, and the peptide used in the present invention may be of any amino acid sequence as mentioned above including deletion, addition, insertion, or substitution as required as long as the peptide includes "the sequence of 18 amino acids exhibiting the four sided structure of the present invention" and retains the function of its own to thereby ensure the function of the peptide chemically modified with PEG.

If necessary, the peptide may also be modified with a molecule other than amino acid as long as the function of the peptide chemically modified with PEG is ensured. For example, the peptide may be modified with a sugar chain, a lipid, or a high molecular weight compound in order to increase in vivo stability, and/or by a sugar chain, a lipid, or a high molecular weight compound in order to suppress the recognition of the peptide by an antigen presenting cell. To be more specific, the peptide may be modified with mannose or cholesterol, and such modified peptides are also comprehended in the peptide used in the present invention.

The peptide used in the present invention does not contain any acidic hydrophilic amino acid, and this feature is particularly useful when the peptide is to be modified. To be more specific, such peptide can be designed to include no acidic amino acid and to include carboxyl group only at its C terminal, and when the peptide is modified by utilizing a reaction depending on the carboxyl group, a site specific modification of the C terminal is enabled.

When the peptide used in the present invention is to be site-specifically modified by utilizing thiol group in cysteine residue, it is advantageous to design the peptide so that only one cysteine is present in the amino acid sequence, and preferably, so that the cysteine is added at the N terminal or C terminal of the peptide because selective modification of the peptide is enabled, for example, in the modification with polyethylene glycol according to the present invention.

By the way, amino acids are categorized by the type and nature of their side chain molecules, and the categorization which may serve an important index in elucidating higher order structure of the peptide is the categorization based on the polarity of the side chain molecule. To be more specific, the amino acids are categorized as described below.
(1) Hydrophobic amino acid: glycine, alanine, valine, leucine, isoleucine, methionine, phenylalanine, tyrosine, cysteine, tryptophan, and proline
(2) Acidic hydrophilic amino acid: aspartic acid, and glutamic acid
(3) Neutral hydrophilic amino acid: serine, threonine, glutamine, and asparagine
(4) Basic hydrophilic amino acid: arginine, lysine, and histidine

Accordingly, in the peptide used in the present invention, amino acids which belong to the same category are mutually replaceable as long as the requirement that "the amino acid sequence of 18 amino acids exhibits the four sided structure of the present invention" is fulfilled. For example, isoleucine and leucine, valine and leucine, tyrosine and phenylalanine, tryptophan and leucine, asparagine and glutamine, serine and threonine, arginine and lysine, and histidine and lysine are mutually replaceable.

In the case of histidine which is categorized as a member of basic hydrophilic amino acids, it is only weakly charged under particular conditions, for example, under the physiological conditions, and it shares the nature similar to that of a neutral hydrophilic amino acid, and therefore, histidine is not only replaceable with a basic hydrophilic amino acid, but also with a neutral hydrophilic amino acid.

In the meanwhile, the amino acids which belong to different categories are also mutually replaceable as long as the requirement that "the amino acid sequence of 18 amino acids exhibits the four sided structure of the present invention" is fulfilled.

Typical examples of such replacements are as described below.
(1) Replacement between a hydrophobic amino acid (neutral) and a neutral hydrophilic amino acid (which is equivalent to replacement between arbitrary neutral amino acids)
   Examples: leucine and threonine; leucine and serine; valine and serine; and tyrosine and serine.
(2) Replacement between a hydrophobic amino acid (neutral) and a basic hydrophilic amino acid (which is a replacement between opposites, namely, hydrophobic/hydrophilic and neutral/basic amino acids, and which is equivalent to the replacement between any amino acids other than acidic hydrophilic amino acids)
   Examples: alanine and arginine; and tyrosine and arginine.
(3) Replacement between a neutral hydrophilic amino acid and a basic hydrophilic amino acid (which is equivalent to the replacement between any hydrophilic amino acids other than acidic hydrophilic amino acids)
   Examples: serine and arginine; and glutamine and arginine.

Furthermore, two or more of the amino acid replacements as described above may be combined as long as the requirement that "the amino acid sequence of 18 amino acids exhibits the four sided structure of the present invention" is fulfilled. An exemplary combination of the amino acid replacements between the amino acids of the same category is the replacement of isoleucine with leucine combined with the replacement of valine with leucine. An exemplary combination of the amino acid replacements between the amino acids of different categories is the replacement of tyrosine with serine combined with the replacement of serine with leucine. An exemplary combination of the amino acid replacement between the amino acids of the same category and the amino acid replacements between the amino acids of different categories is the replacement of isoleucine with leucine combined with the replacement of leucine with threonine. The number of the amino acid replacements that may be combined is not limited as long as the requirement that "the amino acid sequence of 18 amino acids exhibits the four sided structure of the present invention" is fulfilled. However, the number of amino acid replacements combined is preferably 3 or less per 18 amino acids.

For example, Examples 12 and 13 demonstrate that the peptide used in the present invention can include amino acid replacements as long as the requirement that "the amino acid sequence of 18 amino acids exhibits the four sided structure of the present invention" is fulfilled.

A typical peptide used in the present invention is a peptide having any of the amino acid sequences of SEQ ID NO: 1 to SEQ ID NO: 24, and the peptide used in the present invention is preferably a peptide having the amino acid sequence of SEQ ID NO: 16 or SEQ ID NO: 19.

The peptide used in the present invention is a peptide which has an ability of binding to a nucleic acid and an ability of introducing the nucleic acid into a cell, and which also has a specific affinity for phosphatidyl serine.

The amino acids constituting the peptide may be either L- or D-amino acids, and may be amino acids other than typical amino acids or synthetic, modified amino acids as long as they substantially share common nature with the natural amino acids. Exemplary such amino acids include hydroxyproline, homoserine, and methylcysteine.

The present invention provides the peptide chemically modified with PEG that is obtained by modifying the peptide including the amino acid sequence comprising 18 amino acids as described above (the peptide used in the present invention) with PEG, and a complex formed of the peptide chemically modified with PEG and the substance which binds to the peptide (the peptide-binding substance) is also within the scope of the present invention.

The second aspect of the present invention is the complex formed of the peptide chemically modified with PEG and the substance which binds to the peptide (the peptide-binding substance).

Such "complex" may be an aggregate, mixture or composition comprising the peptide used in the present invention, PEG, and the peptide-binding substance.

The site in the peptide used in the present invention to which PEG and/or the peptide-binding substance is to be bonded is not particularly limited as long as the function of the peptide is not impaired.

The results of Example 30 reveal that a complex (such as aggregate, mixture, composition, or the like) is formed between the peptide used in the present invention, PEG, and the peptide-binding substance.

As described above, the "peptide-binding substance" constituting the complex of the present invention may be a nucleic acid, an acidic high molecular weight compound such as acidic protein, or a physiologically active low molecular weight compound having a negatively charged side chain.

Examples of "acidic high molecular weight compounds such as acidic protein" include proteins which are rich in acidic amino acids and which are negatively charged (for example, albumin), and high molecular weight compounds other than proteins wherein the entire molecule is negatively charged (for example, heparin and hyaluronic acid).

Examples of "low molecular weight compounds having a negatively charged side chain" include a low molecular weight compound having phosphate group or the like on its side chain such as phosphorylated acyclovir.

The "nucleic acid" includes a nucleoside, a nucleotide, an oligonucleotide or a polynucleotide comprising two or more nucleotides, a DNA, an RNA, a derivative thereof, a modification thereof, and an analog thereof.

The "nucleic acid derivative" includes a nucleic acid wherein some of the atoms constituting the nucleic acid have been replaced with other atoms. An example of such "nucleic acid derivative" is the PS form wherein one of the oxygen atoms in the phosphodiester bond moiety has been replaced with sulfur atom.

The "modified nucleic acid" includes a nucleic acid wherein some of the atoms constituting the nucleic acid have been replaced with other atomic group or some of the atoms constituting the nucleic acid have other atomic group added thereto. Examples of such "modified nucleic acid" are the one wherein the carbon atom located at 2' position of the pentose moiety in the nucleic acid has methoxy group (-O-CH₃) added thereto, and those wherein the nucleic acid sequence has a sugar, a phospholipid, or polyethylene glycol added thereto in some part thereof.

The "nucleic acid analog" includes a molecule which has a backbone entirely different from that of a nucleic acid while the molecule retains the function expected from the nucleic acid. An example of such "nucleic acid analog" is a peptide nucleic acid (PNA). In this respect, the nucleic acid also comprehends therein a DNA or an RNA which is a polynucleotide that increases or decreases the amount of particular protein expressed in the body, or regulates the expression of the function of particular factor in the body; a derivative, a modification, or an analog of such DNA or RNA; a combination of such derivative, modification and analog; and a mixture or chimera of such derivative, modification, or analog.

Furthermore, the nucleic acid as described above may be a single stranded nucleic acid or a nucleic acid of two or more strands, and may be the one bound to a carrier. For example, the nucleic acid may be the DNA coding for a protein, a plasmid wherein an expression-regulating unit has been linked to such DNA, an antisense oligonucleotide, a double-stranded nucleic acid serving as a decoy (hereinafter referred to as decoy), an aptamer, a ribozyme, or an siRNA.

The "particular protein" or the "particular factor" used herein designates a protein or a factor whose amount expressed is to be increased or decreased, or whose expression is to be regulated by the nucleic acid. Such protein and factor may be either the one found in a living body or the one not found in a living body.

The "ability of binding to a peptide-binding substance" can be assayed, when explained by using a nucleic acid as an example, by subjecting a mixture of the nucleic acid and the peptide used in the present invention to electrophoresis, and detecting the image of the stained nucleic acid. For example, when the nucleic acid is not electrophoresed and the stained image of the nucleic acid is not detected, or when the stained image detected is in the region where distance of the migration of the stained image is small compared to the stained image of the nucleic acid alone, the peptide can be determined to have "the ability of binding to a peptide-binding substance". Illustrative procedure of such assay will be described in Example 8.

The "ability of introducing a peptide-binding substance into a cell" can be measured, when explained by using a nucleic acid as an example, by observing the cell under a fluorescence microscope using a fluorescent-labeled nucleic acid, or by using a plasmid which expresses a reporter gene and measuring the reporter protein expressed by the cell. The ability can also be measured by using the pharmacological action resulting from the expression of the reporter protein as the index. Typical reporters include firefly luciferase, β-galactosidase, and HSV-tk, and illustrative procedure will be described in Example 9. When the amount of the firefly luciferase expressed by the cell is measured by such procedure, fluorescent count per 1 mg of protein per 1 second is measured. When the fluorescent count is 10,000 or more, the reporter gene is determined to have been introduced into the cell, and the peptide is determined to have the "ability of introducing a peptide-binding substance into a cell".

It is to be noted that, in the present invention, the "introduction in (in/into/to) the cell" designates the same situation as the "introduction into the interior of the cell".

In the present invention, "the interior of the cell" designates the units constituting the cell and their interior. For example, included in "the interior of the cell" are the inside of the phospholipid bilayer constituting the contour of the cell, the space between the two layers of the phospholipid bilayer, as well as cytoplasm, organella, nucleus, and their interior.

"Specific affinity" means that a peptide exhibits some specific interaction or other, for example, binding, formation of complex, mutual recognition of the molecule, tendency of moving in a particular direction or being collected in a particular direction, change of molecular configuration, or mutual reaction. For example, a peptide is determined to have a specific affinity if the peptide exhibits affinity in the presence of serum albumin.

Even if a peptide interacts with a particular substance, the interaction is generally nonspecific if the interaction disappears in the presence of other peptides or proteins. To be more specific, a nonspecific interaction disappears in the presence of a large amount of albumin or other protein. Accordingly, a peptide which exhibits affinity for phosphatidyl serine in the absence of serum albumin but fails to exhibit affinity for phosphatidyl serine in the presence of serum albumin is nonspecific with regard to the interaction with phosphatidyl serine, and the peptide will exhibit no affinity for phosphatidyl serine in a living body.

On the other hand, when the interaction of a peptide with the particular substance does not disappear in the presence of other peptides or proteins, the interaction can be deemed specific, and the peptide can be regarded to have a "specific affinity".

A "carrier" is a substance which binds to or incorporates a drug or the like for its delivery. While the carrier is not limited to any particular type as long as it has such function, the carrier is preferably the one comprising a lipid or a high molecular weight compound, and the preferable examples include liposomes, dendrimers, and nanoparticles. Combination of the carrier and the drug is also not limited, while the combination should be the one capable of holding the drug in a stable manner. In this point of view, an electrostatically repelling combination should preferably be avoided, and an exemplary preferable combination is use of positively charged doxorubicin with a neutral or negatively charged liposome.

Use of the carrier modified with the peptide chemically modified with PEG of the present invention is particularly favorable since it has targetability to a specific site in addition to an improved in vivo kinetics. With regard to the peptide chemically modified with PEG, the activated PEG used in the chemical PEG modification is not limited to any particular type as long as formation of the complex with the carrier is enabled. However, when the carrier contains a phospholipid as its constituent, the activated PEG is preferably the one having a phospholipid bonded thereto. The ratio of the number of molecules of the PEG not modified by the peptide to the number of molecules of the peptide chemically modified with PEG of the present invention is not particularly limited as long as in vivo kinetics of the carrier modified with the peptide chemically modified with PEG is improved. However, when the carrier is a liposome, the ratio is preferably 1:1 to 1:0.001, more preferably 1:0.3 to 1:0.01, and most preferably 1:0. 2 to 1:0. 02.

Next, the properties and characters of the peptide used in the present invention, as well as the improved properties and characters of the peptide chemically modified with PEG of the present invention are described by referring to the Examples.

It is to be noted that the improved properties and characters of the peptide chemically modified with PEG of the present invention include the properties and characters of the complex of the peptide chemically modified with PEG of the present invention and the peptide-binding substance and the properties and characters of the carrier which has been modified with the peptide chemically modified with PEG of the present invention.

The peptide used in the present invention (namely, the peptide including the amino acid sequence comprising 18 amino acids that is not modified with PEG) and the peptide chemically modified with PEG of the present invention (both two peptides alike being hereafter referred to as "the peptide of the present invention") have the ability of binding to a nucleic acid. Preferably, the peptides are each the one provided with the ability of forming a complex with the nucleic acid by substantial integration between the peptide and the nucleic acid without completely compromising any of other abilities of the peptide such as the ability of introducing the nucleic acid into a cell or the affinity for phosphatidyl serine in the presence of serum albumin, and the functions inherent to the nucleic acid as well.

The peptide of the present invention is not limited for its mode of binding to the nucleic acid, and the binding may be accomplished, for example, by electrostatic bonding, hydrophobic bonding, or covalent bonding.

The peptide of the present invention has ability of forming the complex as above to introduce thereby the nucleic acid that has become bonded to the peptide into the interior of a cell. Preferably, the peptide of the present invention has ability of introducing the nucleic acid into a cell without causing decomposition of the nucleic acid and with the desired function of the nucleic acid maintained.

In the nucleic acid-introducing ability measurement of Example 9, the nucleic acid-introducing ability of the peptide used in the present invention, as being determined as the fluorescent count per 1 mg of protein per 1 second, has a specific measured value of at least 10,000. Preferably, the peptide used in the present invention is a peptide which exhibits a nucleic acid-introducing ability of at least 100,000, more preferably of at least 1,000,000.

Since the peptide of the present invention is provided with the features as described above, the nucleic acid introduced by using the peptide is capable of exerting its desired functions in the cell once it is introduced in the cell. For example, the exogenous gene inserted in the plasmid may become expressed in the cell to produce the desired protein, or the antisense oligonucleotide, decoy, aptamer, ribozyme, or the like may suppress production of a particular physiologically active substance. It is to be noted that "the desired protein" not only includes the final active form of the protein but also the precursor for such final form of the protein.

Examples 11 and 24 will illustrate embodiments wherein firefly luciferase gene that has been inserted in a plasmid is introduced in a cell by the peptide used in the present invention, and after its transcription and translation, firefly luciferase is produced and accumulated in the cell. Examples 16 and 25 will illustrate embodiments wherein thymidine kinase (hereinafter abbreviated as HSV-tk) gene from herpes simplex virus that has been inserted in the plasmid is introduced in a tumor cell by the peptide used in the present invention, and after its transcription and translation, HSV-tk is produced and accumulated in the tumor cell to thereby enhance ganciclovir sensitivity of the tumor cell and exert pharmacological action (anti-tumor action).

Furthermore, a preferred embodiment of the peptide of the present invention is a peptide which exhibits specific affinity for phosphatidyl serine in the presence serum albumin, and which does not exhibits any affinity for phospholipids other than phosphatidyl serine, for example, phosphatidyl choline.

The interaction between the preferred embodiment of the peptide of the present invention which has no affinity for a phospholipid other than phosphatidyl serine and the phosphatidyl serine, namely, the binding between the peptide of the present invention and the phosphatidyl serine is not limited to the binding through charge or the binding through non-specific binding, and the binding may also be the binding enabled by the recognition of the molecular structure of the phosphatidyl serine by the peptide.

This is demonstrated, for example, in Example 20 and Example 21 with respect to the peptide used in the present invention.

As described above, phosphatidyl serine is the phospholipid which is included as a component constituting the lipid bilayer forming the surface layer of a cell, and the proportion of the phosphatidyl serine found in the outer layer and the inner layer of the lipid bilayer varies depending on the condition of the cell. To be more specific, phosphatidyl serine is a phospholipid which is believed to increase its proportion in the outer layer of the lipid bilayer in an abnormal cell such as a cell in the inflammatory lesion wherein the cell has been injured, denatured or activated. Therefore, the peptide of the present invention selectively binds to the abnormal cell, for example, the cell in the inflammatory lesion. Phosphatidyl serine is also a phospholipid which provides a "field" in a living body for the blood coagulation reaction to take place, or a mark when macrophage recognizes and eats an apoptotic cell. Therefore, the peptide of the present invention selectively binds to the abnormal cell, for example, to the "field" in a living body where blood coagulation reaction is in progress.

Thus, the peptide of the present invention is characterized by its ability of binding to a nucleic acid, its ability of introducing the nucleic acid to a cell, and its affinity for phosphatidyl serine in the presence of serum albumin.

The peptide of the present invention is preferably a peptide which takes an irregular structure in an aqueous solution containing no solute or only an inorganic salt but which takes the α-helix structure in the presence of a particular substance.

The "particular substance" used herein designates a substance which interacts with the peptide of the present invention to promote the peptide to take the α-helix structure, and an exemplary such substance is an amphipathic substance, for example, a surfactant such as sodium dodecyl sulfate (SDS) or a particular phospholipid such as phosphatidyl serine.

α-helix structure in the higher order structure of a peptide can be generally confirmed by measuring CD spectrum. To be more specific, when α-helix structure is present in the higher order structure of a peptide, mean residue ellipticity in the CD spectroscopy takes the form of "W" which is characteristic to the α-helix structure wherein local minimum is found in two wavelength regions, namely, in the region at the wavelength of 205 to 210 nm and the region at the wavelength of 220 to 225 nm. (See "Optical rotation of proteins" (Experimental methods in biological chemistry 6), Hamaguchi, H. et al., Japan Scientific Societies Press, 1979). It is to be noted that the proportion of the α-helix structure in the higher order structure of the peptide can be calculated by a predetermined calculation method from the mean residue ellipticity that had been measured. Typical examples of such calculation include the method of Chen et al. (Y.H. Chen et al., Biochemistry Vol. 11, 4120 (1972)), the method of Yang et al. (J.T. Yang et al., Anal. Chem., Vol. 91, 13 (1978)), and the method of Woody et al. (R.W. Woody et al., J. Mol. Biol., Vol. 242, 497 (1994)).

The value calculated, however, varies by the method used for the calculation, and therefore, it is recommended that the method employed for the calculation is indicated together with the value calculated.

As described above, the preferred embodiment of the peptide of the present invention takes α-helix structure in the presence of a particular substance. For example, such preferred peptide takes α-helix structure through interaction with an amphipathic substance such as a surfactant or a particular phospholipid on the cell membrane such as phosphatidyl serine. This invites increase in the permeability of the peptide or the substance containing the peptide through cell membrane, and smooth migration of the peptide or the substance containing the peptide into the cell.

To be more specific, the preferred embodiment of the peptide of the present invention does not exhibit α-helix structure in the CD spectroscopy in an aqueous solution containing no solute or in an aqueous solution containing only an inorganic salt at a pH of 5 to 8, but which shows two local minimums in the mean residue ellipticity in the CD spectroscopy in an aqueous solution containing 5mM SDS at a pH of 5 to 8 at two wavelength regions, namely, at the wavelength region of 205 to 210 nm and at the wavelength region of 220 to 225 nm, indicating the α-helix structure.

The peptide is preferably the one wherein the proportion of the α-helix structure in the higher order structure is 25% or more when calculated by the method of Chen et al. More preferably, the peptide is the one wherein the proportion of the α-helix structure is 30% or more, and still more preferably, the one wherein the proportion of the α-helix structure is 35% or more.

Such peptide of the present invention as above is found to take an α-helix structure in an aqueous solution in the presence of an amphipathic substance. In addition, since such peptide has affinity for phosphatidyl serine, such peptide selectively accumulates on the surface of an abnormal cell, for example, on the surface of an injured, denatured, or activated cell, and takes the α-helix structure through interaction with a phosphatidyl serine on the cell membrane, and the peptide is then selectively incorporated in the cell.

The peptide of the present invention is preferably the one which does not form aggregates in the presence of a protein.

For example, in the case of the preferred embodiment of the peptide of the present invention, the peptide will not take an α-helix structure in the absence of an amphipathic substance, and solubility will be maintained in the presence of proteins. As a consequence, the peptide is prevented from forming aggregates, which may cause substantial problem, even when administered to a human, and the risk of the blood vessel occlusion is reduced with an increased safety. Example 26, for example, demonstrates the high safety of the peptide used in the present invention.

It should be noted that the peptide chemically modified with PEG of the present invention is more improved in safety.

Whether or not the peptide of the present invention forms the aggregates to a substantial level in the presence of a protein can be determined by optically measuring turbidity of an aqueous solution of serum albumin containing the peptide, for example, by measuring absorption of the aqueous solution of serum albumin containing the peptide at a wavelength of 340 nm to 660 nm, and in particular, at a wavelength of 600 nm.

The peptide of the present invention is useful as a peptide vector because of its characteristic features that the peptide easily binds to the nucleic acid, that it exhibits high solubility after forming the complex with the nucleic acid, that it is highly capable of introducing the nucleic acid in the cell, that it demonstrates the high safety, and that it has affinity for phosphatidyl serine in the presence of serum albumin enabling its selective incorporation into the abnormal cell.

The "peptide vector" is a peptide which is capable of introducing the desired substance into a cell, its derivative, its modification, or its analog.

The peptide of the present invention also has a characteristic feature that it can prevent decomposition of the nucleic acid by a nuclease. Accordingly, a natural type oligonucleotide or polynucleotide (P=O form) which is easily decomposed by the nuclease in the blood or cell becomes resistant to the decomposition by the nuclease once the peptide of the present invention is bonded to such oligonucleotide or polynucleotide.

Whether or not the peptide imparts the nuclease resistance to the nucleic acid can be determined by allowing the complex of the nucleic acid and the peptide of the present invention to react with the nuclease in a solution containing the nuclease, extracting the nucleic acid, and subjecting the extracted nucleic acid to electrophoresis and detecting the stained image. For example, if the peptide imparts the nucleic acid with the nuclease resistance, the nucleic acid will remain intact and the stained image of the nucleic acid will be obtained. Examples 18 and 19 recite the detailed procedure.

When the peptide of the present invention is used as a peptide vector, a substance capable of binding to the vector, which is preferably a nucleic acid, can be introduced into the cell, and development of the function of the nucleic acid in the cell is realized by such introduction in the cell of the nucleic acid.

Examples 11 to 16 are directed to the embodiments wherein the peptide of the present invention is used to an in vitro introduction of a nucleic acid into the cell, and the firefly luciferase and HSV-tk coded by the nucleic acid are respectively expressed in the cell. Examples 24 and 25 are directed to the embodiments wherein the peptide used in the present invention is adapted to an in vivo introduction of a nucleic acid in the cell, and the firefly luciferase and HSV-tk coded by the nucleic acid are respectively expressed in the cell. In particular, Example 25 is directed to the embodiment wherein the HSV-tk gene that has been introduced in the tumor cell by the peptide used in the present invention is expressed in the tumor cell, and ganciclovir sensitivity of the tumor cell is thereby enhanced to exhibit the pharmacological action (anti-tumor action). The dose of the plasmid used in this Example was 10 µg, and this dose was by far smaller than the dose (150 µg) used in similar pharmacological experiment using the conventional liposome vector (Aoki, K. et al., Hum. Gene Ther., Vol. 8, 1105 (1997)). As will be more illustratively shown in Examples 17 and 18, the peptide used in the present invention increases the stability of the nucleic acid to an extent further than the liposome, and the peptide used in the present invention that has become bonded to the nucleic acid has a stability higher than that of the liposome, and therefore, use of the peptide used in the present invention in introducing a nucleic acid in a cell is useful, and the peptide used in the present invention is excellent for use as a vector in introducing a nucleic acid in a cell.

The peptide chemically modified with PEG of the present invention that includes the peptide of excellent characters as above is more excellent in such characters.

Other exemplary uses of the peptide of the present invention as a vector include use of the peptide in introducing the following substances into the cell to thereby allow development in the cell of the function of the following substances:
a plasmid expressing a reporter protein (green fluorescent protein (GFP), β-galactosidase, etc.);
a plasmid expressing a cytokine (interleukin 2, interferon β, etc.) which exhibits anti-tumor effects;
a plasmid expressing a physiologically active substance (Fas ligand, p53, caspase 3, caspase 8, Bax (Bcl-2-associated X protein), FADD (Fas associated death domain protein), etc.) which induces apoptosis to exert cytotoxic effects;
a plasmid expressing a soluble receptor for a ligand such as TNF-α or interleukin 6, which competitively binds to the ligand to suppress the reaction induced by the ligand, and which can thereby improve the symptom of, for example, chronic articular rheumatism;
a plasmid expressing a peptide/polypeptide which can serve a vaccine to suppress an allergic reaction or a protein such as mite antigen which is an antigenic protein;
a plasmid expressing vascular endothelial growth factor (VEGF) or hepatocyte growth factor (HGF) which has the action of improving the pathological condition of arteriosclerosis obliterans as a circulatory disease or promoting the healing (remodeling) of the injured lesion;
an antisense oligonucleotide or a ribozyme for CDC2 kinase which has the action of suppressing the restenosis after percutaneous transluminal coronary angioplasty (PTCA); and
a decoy for a nucleotide sequence of to which E2F (a transcriptional regulatory factor for a cell cycle regulatory gene) or NFκB (a transcriptional regulatory factor for a cytokine) binds; as well as
a phosphorylated nucleic acid analog in its active form which exhibits an anti-virus action, and the like.

Since the peptide of the present invention has the ability of binding to a nucleic acid, ability of introducing the nucleic acid to a cell, and affinity for phosphatidyl serine in the presence of serum albumin as its characteristic features, the peptide will introduce the nucleic acid at a higher efficiency to a cell wherein a larger amount of phosphatidyl serine has been translocated to the surface. This means that, when a nucleic acid such as a gene or an antisense DNA is to be introduced in a cell for the purpose of treating a disease, the nucleic acid will be selectively introduced in a larger amount to an abnormal cell such as an injured, denatured, or activated inflammatory cell, or to an immunocompetent cell wherein an increased amount of phosphatidyl serine has been translocated to the cell surface. Reduction of the side effects is thereby attained.

For example, when the peptide of the present invention is used as a vector in introducing a nucleic acid to a tumor cell for the purpose of treating a cancer, the nucleic acid is scarcely introduced to a normal cell, while the nucleic acid is introduced to the tumor cell at a high rate. When a nucleic acid is introduced in a cell for the purpose of treating an allergy, the nucleic acid is specifically introduced to a cell wherein allergic reaction has been induced by the immunocompetent cell.

Furthermore, the nucleic acid can be introduced at a higher rate to the desired particular cell or organ by utilizing the capability of the peptide of the present invention "to introduce the nucleic acid at a higher rate to the cell wherein a larger amount of phosphatidyl serine has been translocated to the cell surface", and namely, by preliminarily increasing the amount of the phosphatidyl serine translocated to the surface of the desired particular cell or organ. To be more specific, introduction of the nucleic acid at a higher rate to the desired particular cell or organ can be realized by reacting a reagent with the particular cell or organ to thereby increase the amount of the phosphatidyl serine translocated to the surface of the particular cell or organ by the pharmacological action of the reagent, and thereafter using the peptide of the present invention as a vector.

For example, a therapy is still possible even if a chemotherapeutic treatment of a tumor by sole administration of an anticancer drug failed to achieve sufficient therapeutic effects, and in such a case, the chemotherapeutic agent may be administered to increase the amount of the phosphatidyl serine translocated to the surface of the tumor cell by the pharmacological action of the chemotherapeutic agent, and then, the peptide of the present invention may be used as a vector to thereby realize the highly efficient introduction of a gene, the antisense DNA, or other nucleic acid which exhibits high anti-tumor effects into the tumor cell and utilize the improved therapeutic effects of the nucleic acid.

As described above, the peptide of the present invention readily binds to a nucleic acid and introduces the nucleic acid into the cell. The peptide of the present invention, however, is not only capable of introducing a nucleic acid but also capable of introducing another peptide-binding substance into a cell. As in the case of the binding of the peptide of the present invention with the nucleic acid wherein the mode of the binding is not particularly limited, the mode of the binding is not limited in the case of the binding of the peptide of the present invention with such substance. The mode of the binding between the peptide of the present invention and such substance upon introduction of such substance into the cell is preferably a noncovalent bond, more preferably an electrostatic bond, and most preferably an electrostatic bond established between the positive charge of the peptide of the present invention and the negative charge of the substance. In other words, the present invention includes within its scope an introduction of a peptide-binding substance into a cell based on the mode of the binding as described above.

It is to be noted that, while the properties and characters of the peptide used in the present invention and the peptide chemically modified with PEG of the present invention have been described together, the peptide chemically modified with PEG of the present invention is the peptide used in the present invention which has been chemically modified with the PEG, and it exhibits improved efficiency in incorporating the gene or the drug into the target cell, improved pharmacological activity, reduced toxicity, and other favorable effects compared to the peptide used in the present invention.

More specifically, as evident from the results of Examples 28 and 29 as will be described below, specific activity is not impaired when the peptide used in the present invention is modified with the activated PEG (Example 28), and the introduction rate of the peptide-binding substance increases about three times (Example 29). The usefulness of the present invention is thereby demonstrated.

As evident from the results of Examples 35 to 37 as will be described below, the carrier which is modified with the peptide chemically modified with PEG of the present invention is capable of improving the introduction rate of the drug incorporated in the carrier by 3 to 33 folds (Examples 35 and 36), and extending the survival period of the cancer bearing mouse (Example 37). The usefulness of the present invention is thereby demonstrated.

In the following, it is described how the peptide chemically modified with PEG of the present invention is produced.

The peptide used in the present invention can be produced by chemical synthesis.

For example, the peptide is obtained by a synthesis using an automatic peptide synthesizer (432A, manufactured by Applied Biosystems).

The peptide used in the present invention may also be produced by a genetic engineering means. When the peptide is produced by genetic engineering methods, the desired peptide may be produced by the following steps:
(1) the step of producing a DNA having the nucleotide sequence coding for the amino acid sequence of the peptide;
(2) the step of introducing the DNA in a vector to thereby produce an amplifiable recombinant DNA including the DNA;
(3) the step of transforming a host cell with the recombinant DNA to produce a transformant capable of expressing the peptide; and
(4) the step of cultivating the transformant to produce the peptide, and recovering the peptide from the culture mixture.

The DNA coding for the peptide used in the present invention may be any DNA having the nucleotide sequence which substantially codes for the peptide used in the present invention. As is well known in the art, because of the degeneration of codon, at least one nucleotide in the gene sequence can be replaced with another nucleotide without causing any change in the amino acid sequence of the peptide coded by the gene sequence. Therefore, the DNA may have a nucleotide sequence wherein at least one nucleotide in the nucleotide sequence has been replaced on the bases of the degeneration of the genetic code. To be more specific, when the peptide used in the present invention is produced by using genetic engineering methods, the peptide may have a nucleotide sequence wherein at least one nucleotide has been replaced so that the codon will be the one frequently found in a particular host cell. In addition, the DNA may be a recombinant DNA, such as a plasmid or an expression vector.

Exemplary DNAs of SEQ ID NO: 28 to SEQ ID NO: 30 coding for the peptides of SEQ ID NO: 1, SEQ ID NO: 16, and SEQ ID NO: 19 are shown.

The step of obtaining the DNA having a nucleotide sequence coding for the amino acid sequence of the peptide may be accomplished by the synthesis using an automatic nucleic acid synthesizer.

The step of incorporating the DNA in the vector to obtain an amplifiable recombinant DNA including the DNA, and the step of transforming the host cell by the recombinant DNA to obtain a transformant which is capable of expressing the peptide may be accomplished by the genetic engineering methods generally used in the art as described in a book (for example, Molecular Cloning: a laboratory manual, Second edition, T. Maniatis et al., Cold Spring Harbor Laboratory Press (1989)). It is to be noted that, since the peptide used in the present invention can be designed as a peptide including no methionine, the peptide can be obtained by producing a peptide wherein a plurality of the peptides of the present invention are ligated by the intervening methionine by genetic engineering methods, and cleaving the peptide thus produced with cyanogen bromide.

The peptide produced may be purified, isolated, and recovered by referring to methods described in various articles and books (for example, "Experiments in Biochemistry: A New Lecture Series: Protein I" (the Japanese Biochemical Society, ed., Tokyo Kagaku Dozin, 1990), "Kagaku, Special edition, 102: high performance liquid chromatography of proteins and peptides" (N. Ui et al., ed., Kagaku-Dojin, 1985)). To be more specific, the peptide produced may be obtained in its pure form by using at least one of the procedures selected from demineralization, concentration, salting out, ultrafiltration, ion exchange chromatography, reversed phase chromatography, isoelectric chromatography, affinity chromatography, and gel permeation.

The peptide chemically modified with PEG of the present invention is produced by chemically modifying the peptide used in the present invention produced by the procedure as described above with the PEG as described above.

The conditions of the chemical modification with the PEG is not particularly limited, any adequate process may be selected from those known to those skilled in the art.

Typically, the solution of the peptide in an adequate solvent and the solution of the activated PEG in an adequate solvent are mixed, and the mixture is allowed to react at about 1 to about 40°C for about 1 minute to about 24 hours.

The peptide chemically modified with PEG may also be purified after the chemical modification with the PEG by using the process and conditions commonly used in the art.

In the production process as described above, the activated PEG is used at an amount in molar ratio to the amount of the peptide used in the present invention of 0. 0001 to 10000, and preferably 0.01 to 100, and most preferably 0.1 to 10. When the activated PEG is used at an amount in such a range, the peptide chemically modified with PEG will retain the activity of the original peptide while acquiring increased solubility, reduced antigenicity, reduced toxicity, and other favorable characters after the reaction of the activated PEG with the peptide.

The PEG moiety in the peptide chemically modified with PEG may have an average molecular weight of about 200 Da to about 100,000 Da, preferably about 1,000 Da to about 50,000 Da, and more preferably about 2,000 Da to about 20,000 Da.

When the average molecular weight of the PEG moiety is within such range, the peptide modified with PEG will retain the activity of the original peptide while acquiring increased solubility, reduced antigenicity, reduced toxicity, and other favorable characters.

Next, the process of producing the complex of the peptide chemically modified with PEG of the present invention and the peptide-binding substance is described.

The complex is produced by using the peptide used in the present invention, the activated PEG, and the peptide-binding substance as described above, by the process comprising the steps of:
I)a) reacting the peptide containing the sequence comprising 18 amino acids with the activated polyethylene glycol (PEG), and
   b) reacting the peptide chemically modified with PEG obtained in the above step a) with the substance which binds to the peptide; or, by the process comprising the steps of:
II)a) reacting the peptide containing the sequence comprising 18 amino acids with the peptide-binding substance, and
   b) reacting the reaction product of the peptide and the peptide-binding substance with the activated PEG.

The method II) is preferable since unnecessary PEG modification of the peptide can be avoided by preliminarily forming the reaction product of the peptide with the peptide-binding substance, and modifying the reaction product with the PEG.

By employing the method II), unnecessary PEG modification such as coverage in the PEG modification of the site required for the peptide functioning (coverage of the active center of the peptide), peptide structure change that may hamper the approach of the peptide to the target, and other incidents that may lead to the reduced specific activity can be avoided to further improve the usefulness of the PEG modification.

The conditions used for the production of the peptide chemically modified with PEG of the present invention may be used for the reaction conditions of the step a) of method I), and the reaction conditions similar to those of method II) a) as will be described below may be used for the reaction conditions of step b) of method I).

The reaction conditions used in step a) of method II) is not particularly limited, and any conditions enabling the formation of the reaction product may be selected. A typical condition is the condition wherein the peptide and the peptide-binding substance that have been respectively dissolved in appropriate solvents are mixed at about 1 to about 40°C for about 1 minute to about 24 hours.

The reaction conditions used in step b) of method II) is not particularly limited, and any condition enabling the formation of the complex may be selected. Typical reaction conditions are those used for chemical modification with PEG of the peptide used in the present invention.

The reaction conditions are more specifically described in Example 27.

In such production methods, the peptide used in the present invention, the activated PEG, and the peptide-binding substance may be used at any arbitrary amount and ratio as determined by considering the application of the complex, and the properties, characters, pharmacological activity, safety, and the like required for the complex.

Specifically, in step a) of method II), the peptide-binding substance is used such that the ratio of the number of positive charges (+) of the peptide to the number of negative charges (-) of the peptide-binding substance (+/ratio) is 2 or more, preferably 3 or more. The ratio, on the other hand, is preferably up to 100, and more preferably up to 50. When the ratio is within such range, the peptide can form a complex with the peptide-binding substance.

The activated PEG is used in step b) of method II) at an amount in the molar ratio to the peptide used in the present invention of 0.0001 to 10000, preferably 0.01 to 100, and more preferably 0.1 to 10. When the activated PEG is used at an amount in such a range, the PEG-modified peptide will retain the activity of the original peptide after the reaction between the activated PEG and the peptide, and the PEG-modified peptide will be imparted with favorable properties such as improved solubility, reduced antigenicity, and reduced toxicity.

It goes without saying that the complex of the peptide chemically modified with PEG of the present invention with the peptide-binding substance, the mixture, composition and aggregates of the three components, and the like are all within the scope of the present invention. The results of Example 30 indicate that a complex (aggregate, mixture, composition, or the like) is formed by the peptide used in the present invention, the PEG, and the peptide-binding substance are present.

### EXAMPLES

The present invention is described in further detail by referring to the following Examples which are presented for the purpose of illustration and which by no means limit the scope of the invention. The abbreviations used in the following description are those customarily used in the field of the art.

### <Example 1> Chemical synthesis of the peptide

Peptides having the amino acid sequences of SEQ ID NO: 1 to SEQ ID NO: 27 were synthesized by solid phase synthesis by using an automatic peptide synthesizer (432A, manufactured by Applied Biosystems). It is to be noted that, in synthesizing a peptide having a length of 30 residues or longer, synthesis was suspended without deprotecting the 25th amino acid residue, and the synthesis was accomplished by resuming the synthesis after providing the synthesizer with the amino acid columns of 26th and remaining residues. Unless otherwise noted, the synthesis was conducted in accordance with the manufacturer's manual. The peptide was cleaved, deprotected, precipitated in ether, stripped of the ether, dissolved in distilled water, and lyophilized. Next, the peptides of SEQ ID NO: 1 to SEQ ID NO: 26 were dissolved in 20% acetonitrile aqueous solution containing 10 mM HCl, and the peptide of SEQ ID NO: 27 was dissolved in 15% acetonitrile/15% isopropanol aqueous solution containing 10 mM HCl. By using C18 column (CAPCELLPAK C18AG120, manufactured by Shiseido) and high performance liquid chromatography (625 LC System, manufactured by Waters), the peptides of SEQ ID NO: 1 to SEQ ID NO: 26 were purified so that single peak is obtained in linear concentration gradient of 20% to 70% acetonitrile aqueous solution containing 10 mM HCl, and the peptide of SEQ ID NO: 27 was purified so that single peak is obtained in linear concentration gradient of 15% to 50% acetonitrile/15% to 50% isopropanol aqueous solution containing 10 mM HCl. The thus purified peptides were lyophilized, dissolved in distilled water, and stored after frozen.

The peptides were produced at a yield of 30 mg to 40 mg, respectively.

### <Example 2> Assay of peptide (1)

The resulting synthetic peptides were evaluated for their molecular weight by mass spectroscopy using MALDI-TOFMS mass spectrometer (VoyagerDE-STR, manufactured by PE Biosystems) to thereby confirm that the resulting peptides were the desired peptides. Unless otherwise noted, the spectroscopy was conducted in accordance with the manufacturer's manual. The procedure was as summarized below.

First, α-cyano-4-hydroxycinnamic acid (CHCA) was dissolved in 0.1 vol% TFA/50 vol% acetonitrile/pure water to prepare a 10 mg/mL matrix solution. Then, 0.5 µL of aqueous solution (10 pmol/µL) of the peptide produced by the procedure described in Example 1 and 0.5 µL of the matrix solution were mixed on a sample plate, and the mixture was dried for crystallization of the sample. The analysis was conducted under the following conditions.
Measurement mode: Linear, positive
Calibration: external standard method (Note that the standards were (i) Angiotensin I, (ii) ACTH (1-17clip), (iii) ACTH (18-39clip), (iv) ACTH (7-38clip), and (v) Insulin (bovine)).

It was then confirmed that all of the synthetic and purified peptides were consistent with the theoretical molecular weight.

### <Example 3> Assay of peptide (2)

The solutions of the synthetic peptide produced in Example 1 were determined for their concentration by analyzing amino acid composition by ninhydrine method.

The samples were exsiccated in a glass test tube, and after adding 100 µL of 6N HCl and evacuating and sealing the test tube, hydrolysis was allowed to proceed at 110°C for 22 hours. The samples were then exsiccated, dissolved in pure water, and analyzed in an amino acid analyzer (L-8500, manufactured by HITACHI). The peptide aqueous solutions had a concentration of 7 to 10 mg/mL.

### <Example 4> Measurement of turbidity in BSA

Aqueous solutions were prepared so that the resulting solution had a bovine serum albumin (BSA) concentration of 1% and the peptide concentration of 50 µM. The solutions were evaluated for their absorbance at a wavelength of 600 nm by using a spectrophotometer (DU640, manufactured by Beckman). The results are shown in Table 1. No sample exhibited an absorbance that exceeded 0.1.

**Table 1**

| SEQ ID NO: | OD 600 |
|---|---|
| SEQ ID NO: 1 | 0.03 |
| SEQ ID NO: 2 | 0.01> |
| SEQ ID NO: 3 | 0.01> |
| SEQ ID NO: 5 | 0.01> |
| SEQ ID NO: 6 | 0.01> |
| SEQ ID NO: 7 | 0.01> |
| SEQ ID NO: 8 | 0.01> |
| SEQ ID NO: 10 | 0.01> |
| SEQ ID NO: 11 | 0.01> |
| SEQ ID NO: 15 | 0.01> |
| SEQ ID NO: 16 | 0.01> |
| SEQ ID NO: 17 | 0.01> |
| SEQ ID NO: 19 | 0.01> |
| SEQ ID NO: 23 | 0.01> |

### <Example 5> Measurement of CD spectrum

The peptide dissolved in 10 mM phosphate buffer (pH = 7) containing 50 mM NaCl was evaluated for its CD spectrum in the presence and in the absence of 5 mM SDS by using a circular dichroism spectrophotometer (J-500A, manufactured by JASCO). The cell length was 1 mm. The measurement was conducted at 35°C for 6 times in total.

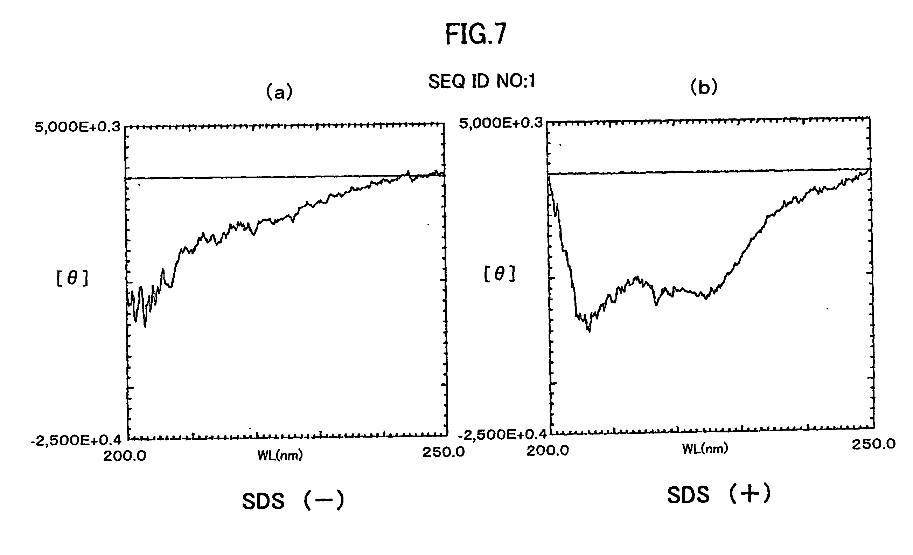

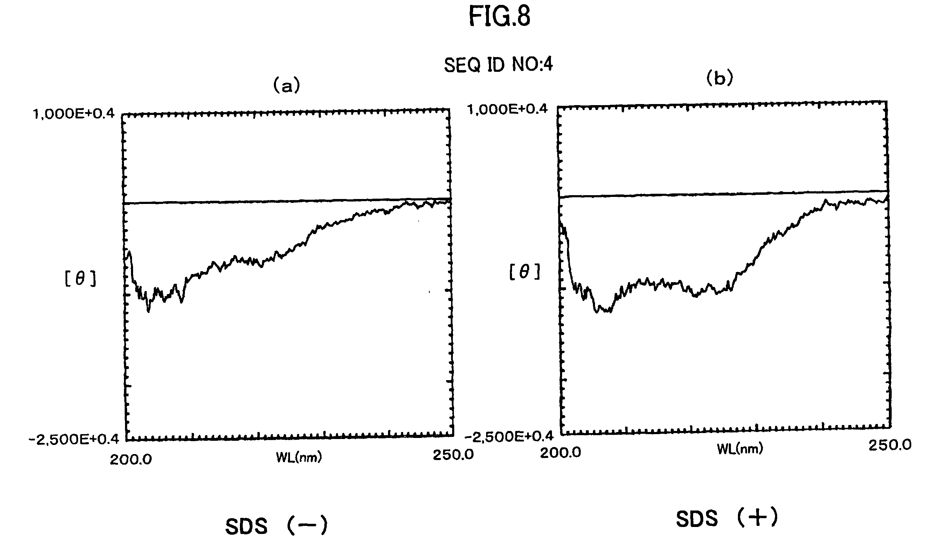

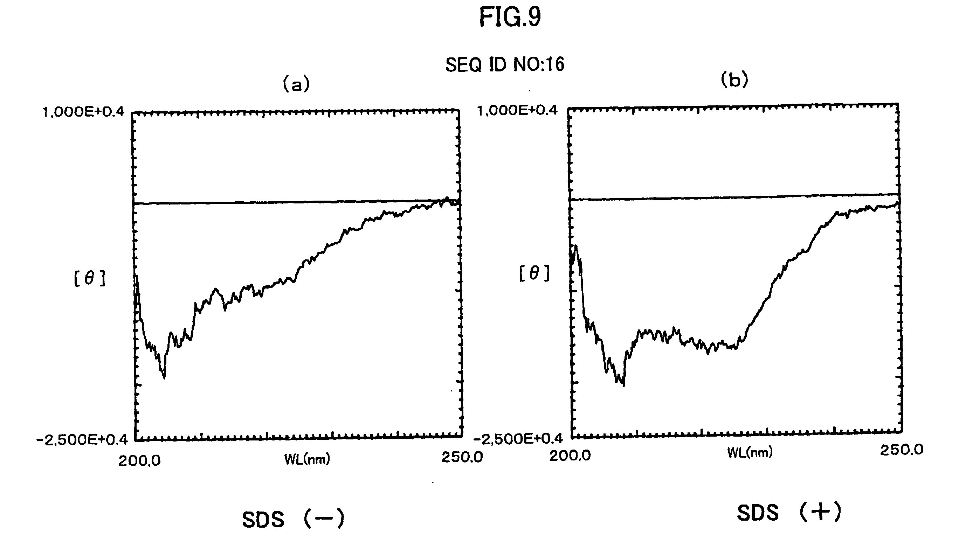

FIGs. 7 to 9 are respectively views showing mean residue ellipticity in CD spectroscopy of the peptide of SEQ ID NO: 1, the peptide of SEQ ID NO: 4 and the peptide of SEQ ID NO: 16. As evident in FIGS. 7 to 9, the mean residue ellipticity obtained in the presence of SDS was the so called W curve and local minimums were found in the areas at the wavelength of 205 to 210 nm and 220 to 225 nm, and α-helix structure was thereby confirmed.

In other words, it was revealed that the peptides of SEQ ID NO: 1, SEQ ID NO: 4, and SEQ ID NO: 16 showing the gene-introducing ability are in α-helix structure in the presence of SDS while they do not take α-helix structure in an aqueous solution solely containing an inorganic salt.

### <Example 6> Synthesis of oligonucleotide

Unlabeled 21mer oligonucleotide was prepared by purification using OPC column (manufactured by Applied Biosystems). FITC-labeled 21mer oligonucleotide was prepared by HPLC using reverse phase chromatography. Synthesis of the oligonucleotide was consigned to Sawady Technology Co., Ltd.

### <Example 7> Preparation of plasmid

For the expression plasmid including firefly luciferase gene as the reporter gene, there were used a commercially available plasmid (pGL3-Control Vector, manufactured by Promega) wherein firefly luciferase gene had been incorporated under SV40 early promoter, a plasmid (pCMV-Luc(F)) wherein firefly luciferase gene had been incorporated under CMV early promoter, and a plasmid (pEF-Luc) wherein firefly luciferase gene had been incorporated under EF-1 α promoter. For the expression plasmid including HSV-tk gene as the reporter gene, there were used a plasmid (pEF-tk) wherein HSV-tk gene had been incorporated under EF-1 α promoter, and a plasmid (pCMV-tk) wherein HSV-tk gene had been incorporated under CMV early promoter. These plasmids and pUC119 and pBR322 which are respectively a universal plasmid were amplified in E. coli when necessary, and purified by a known method before use.

### <Example 8> Evaluation of nucleic acid-binding ability

### (1) Evaluation of binding ability to oligonucleotide

The 21mer oligonucleotide prepared by the procedure described in Example 6 (final concentration, 6.7 µM) and the peptide prepared by the procedure described in Examples 1 to 3 were mixed in 20 mM Tris-HCl buffer (pH = 7.2) containing 150 mM NaCl at a charge ratio (+/- ratio) of 0 to 10, and the mixture was allowed to stand at 37°C for 30 minutes. Next, 7.5 µL of this solution was mixed with an equal amount of Tris-borate buffer (pH = 8.2) containing 80% formamide, and electrophoresis was conducted on 25% polyacrylamide gel containing 7M urea. After the electrophoresis, the gel was stained with 5% aqueous solution of Stains all (manufactured by Funakoshi) containing 50% formamide and washed with water to thereby determine the binding of the oligonucleotide and the peptide.

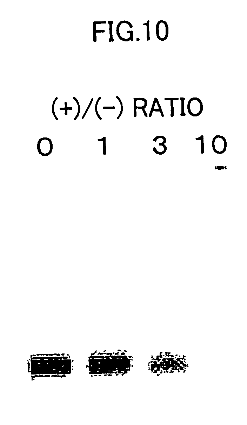

FIG. 10 shows the electropherogram for the peptide of SEQ ID NO: 1. The +/- ratio (charge ratio) indicated in FIG. 10 is the ratio of the number (+) of the positively-charged groups in the peptide to the number (-) of negatively-charged groups in the nucleic acid. As evident in FIG. 10, the amount of oligonucleotide that became bound to the peptide increased with the increase in the charge ratio (+/- ratio), and the oligonucleotide was fully bound to the peptide at a charge ratio (+/- ratio) of 10. The peptides of SEQ ID NO: 2 to SEQ ID NO: 24 were also found to bind at a charge ratio (+/- ratio) of 10.

### (2) Evaluation of binding ability to plasmid

The plasmid prepared by the procedure described in Example 7 (pUC119; final concentration, 20 µg/mL) and the peptides prepared by the procedure described in Examples 1 to 3 were mixed in 20 mM Tris HCl buffer (pH = 7.2) containing 150 mM NaCl at a charge ratio (+/- ratio) of 0 to 3, and the mixture was allowed to stand at 37°C for 30 minutes. Electrophoresis was then conducted on 1% agarose gel to thereby determine the binding of the plasmid and the peptide.

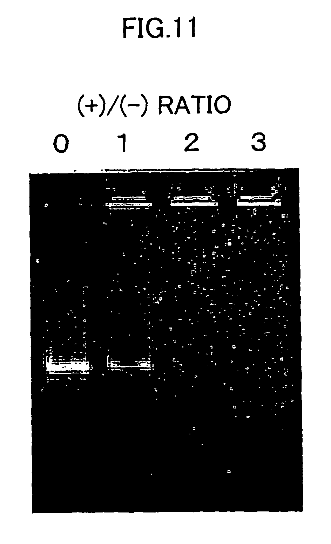

FIG. 11 shows the electropherogram for the peptide of SEQ ID NO: 1. The +/- ratio (charge ratio) indicated in FIG. 11 is the ratio of the number (+) of the positively-charged groups in the peptide to the number (-) of negatively-charged groups in the plasmid. As evident in FIG. 11, the amount of plasmid that became bound to the peptide increased with the increase in the charge ratio (+/- ratio), and the plasmid was fully bound to the peptide at a charge ratio (+/- ratio) of 3. The peptides of SEQ ID NO: 2 to SEQ ID NO: 24 were also found to bind at a charge ratio (+/- ratio) of 3.

### <Example 9> Evaluation of nucleic acid-introducing ability (1)

A cell line established from monkey kidney (Vero cell) and a human bladder cancer cell (T24 cell) purchased from American Type Culture Collection (ATCC) and human lung cancer cell (A549 cell) purchased from Dainippon Pharmaceutical were inoculated on a 24 well culture plate (manufactured by NALGEN NUNC) at 1 x 10⁵ cells/well. Vero cell and A549 cell were cultivated in DMEM (manufactured by Life Technologies Oriental) supplemented with 10% fetal bovine serum (hereinafter referred to as FBS, manufactured by NICHIREI), and T24 cell was cultivated in McCoy' s 5a (manufactured by Life Technologies Oriental) supplemented with 10% FBS in 5% CO₂ atmosphere at 37°C for 24 hours. After removing the medium, opti-MEM (manufactured by Life Technologies Oriental) prepared to have the concentration of the luciferase-expressing plasmid prepared in Example 7 of 1 µg/mL and the concentration of the peptide prepared in Example 1 of 1.25, 2.5, or 5 µM was added, and the cells were cultivated for 5 hours. Next, in the case of Vero cell and A549 cell, the culture medium was replaced with DMEM supplemented with 10% FBS, and in the case of T24 cell, the medium was replaced with McCoy's 5a supplemented with 10% FBS, and cultivation was continued in 5% CO₂ atmosphere and at 37°C for another 24 hours. The luciferase activity expressed in the cell was then measured by the method instructed in luciferase assay system (manufactured by Promega). To be more specific, the cells were washed with phosphate-buffered saline (hereinafter referred to as PBS, manufactured by SIGMA), and the cells were lyzed with Passive Lysis Buffer attached to the kit.

20 µL of this cell lysate and 100 µL of the Luciferase Assay Reagent II attached to the kit was added to a fluorescence-measuring plate (Microlite™ 1 plate, manufactured by Dynatech), and after mixing, fluorescence was measured for 1 second by using a multilabel counter (ARVO™ SY1420 MULTILABEL COUNTER, manufactured by Wallac Beltold.

Concentration of the protein in the cell lysate was measured by mixing 8 µL of the cell lysate and 200 µL of the protein assay solution (manufactured by Biorad) with 792 µL of ultrapure water in a disposable cuvette (UV fluorescent cuvette A204X, manufactured by Funakoshi), allowing the mixture to stand for 5 minutes at room temperature, and measuring the absorption at 595 nm with a spectrophotometer (DU640, manufactured by Beckman). Bovine serum albumin (BSA) was used for the standard protein. By using the thus obtained results, count per 1 second per 1 mg of protein of the cell lysate was calculated to thereby use the count as the luciferase activity, and the highest count in the three conditions of different peptide concentration was designated the gene-introducing ability of the peptide.

### <Example 10> Evaluation of nucleic acid-introducing ability (2)

Vero cell was inoculated in the well of a chamber slide (Lab-Tek II chamber slide, size 4 well, manufactured by NALGEN NUNC) at a rate of 1 x 105 cells/well, and the cells were cultivated in DMEM supplemented with 10% FBS in 5% CO₂ atmosphere at 37°C for 24 hours. After removing the medium, opti-MEM containing 300 nM FITC-labeled oligonucleotide prepared in Example 6 and 2.5 µM of the peptide of SEQ ID NO: 1 prepared in Example 1 was added, and the cultivation was continued for 4 hours. The medium was replaced with DMEM supplemented with 10% FBS, and the cultivation was continued in 5% CO₂ atmosphere at 37°C for another 24 hours. After washing the cell with PBS, the cells were fixed by using PBS containing 4% paraformaldehyde, and accumulation of FITC-labeled oligonucleotide in the cell nucleus was confirmed by using a fluorescence microscope (AH-3, manufactured by Olympus). It was then confirmed that FITC-labeled oligonucleotide was accumulated in the cell nucleus. On the other hand, accumulation of FITC-labeled oligonucleotide in the cell was not confirmed in the absence of the peptide of SEQ ID NO: 1.

### <Example 11> Evaluation of nucleic acid-introducing ability into cell of the peptide (1)

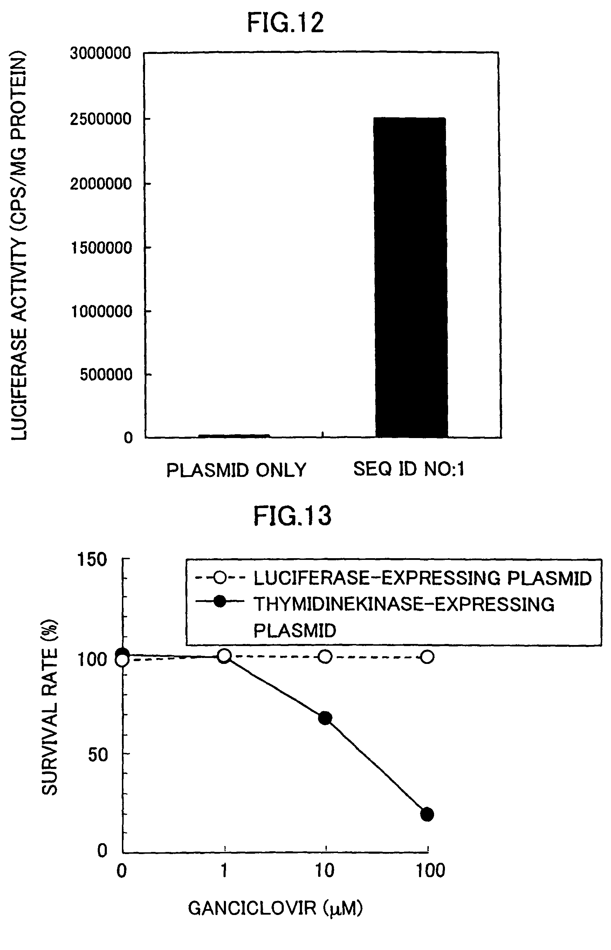

The peptide of SEQ ID NO: 1 was evaluated for its ability of introducing the nucleic acid into a cell by the procedure described in Example 9. FIG. 12 is a view wherein difference in the amount of plasmid introduced into the cell is compared in the presence and absence of the peptide of SEQ ID NO: 1 by using luciferase activity. As shown in FIG. 12, the peptide of SEQ ID NO: 1 was found to have the ability of introducing the nucleic acid into a cell, while the nucleic acid was not introduced in the absence of the peptide of SEQ ID NO: 1.

### <Example 12> Evaluation of nucleic acid-introducing ability into cell of the peptide (2)

The peptides of SEQ ID NO: 2 to SEQ ID NO: 18 having a sequence wherein one or two locations in "the amino acid sequence of 18 amino acids exhibiting the four sided structure of the present invention" have been substituted in the peptide of SEQ ID NO: 1 was evaluated for their ability of introducing the nucleic acid into a cell by the procedure described in Example 9. It was then found that all peptides had the ability of introducing the nucleic acid into a cell.

It is to be noted that the nucleic acid-introducing ability was classified as described below on the bases of the fluorescent count per 1 mg of protein per 1 second (cps/mg protein). The results are listed in Table 2.

| | |
|---|---|
| 10,000 to less than 100,000 | 1+ |
| 100,000 to less than 1,000,000 | 2+ |
| 1,000,000 to less than 10,000,000 | 3+ |
| 10,000,000 to less than 100,000,000 | 4+ |
| 100,000,000 to less than 1,000,000,000 | 5+ |
| 1,000,000,000 or more | 6+ |

It is to be noted that the nucleic acid-introducing ability of SEQ ID NO:1 was 3+ in the above classification.

**Table 2**

| SEQ ID NO: | Nucleic acid-introducing ability |
|---|---|
| SEQ ID NO:2 | 3+ |
| SEQ ID NO:3 | 2+ |
| SEQ ID NO:4 | 4+ |
| SEQ ID NO:5 | 2+ |
| SEQ ID NO:6 | 2+ |
| SEQ ID NO:7 | 2+ |
| SEQ ID NO:8 | 3+ |
| SEQ ID NO:9 | 3+ |
| SEQ ID NO:10 | 3+ |
| SEQ ID NO:11 | 3+ |
| SEQ ID NO:12 | 2+ |
| SEQ ID NO:13 | 3+ |
| SEQ ID NO:14 | 3+ |
| SEQ ID NO:15 | 2+ |
| SEQ ID NO:16 | 4+ |
| SEQ ID NO:17 | 2+ |
| SEQ ID NO:18 | 4+ |
| SEQ ID NO:19 | 4+ |
| SEQ ID NO:20 | 4+ |
| SEQ ID NO:21 | 2+ |
| SEQ ID NO:22 | 3+ |
| SEQ ID NO:23 | 2+ |
| SEQ ID NO:24 | 1+ |

### <Example 13> Evaluation of nucleic acid-introducing ability into cell of the peptide (3)

The peptides of SEQ ID NO: 19 to SEQ ID NO: 22 having a sequence wherein three locations in "the amino acid sequence of 18 amino acids exhibiting the four sided structure of the present invention" had been substituted in the peptide of SEQ ID NO: 1 was evaluated for their ability of introducing the nucleic acid into a cell by the procedure described in Example 9. It was then found that these peptides had the ability of introducing the nucleic acid into a cell.

It is to be noted that the nucleic acid-introducing ability was classified as described in Example 12 on the bases of the fluorescent count per 1 mg of protein per 1 second. The results are listed in Table 2.

### <Example 14> Evaluation of nucleic acid-introducing ability into cell of the peptide (4)

The peptides of SEQ ID NO: 23 and SEQ ID NO: 24 which are respectively a deletion mutant of the peptides of SEQ ID NO: 1 and SEQ ID NO: 4 were prepared, and these peptides were evaluated for their ability of introducing the nucleic acid into a cell by the procedure described in Example 9. It was then found that all of the peptides had the ability of introducing the nucleic acid into a cell.

It is to be noted that the nucleic acid-introducing ability was classified as described in Example 12 on the bases of the fluorescent count per 1 mg of protein per 1 second. The results are listed in Table 2.

### <Example 15> Evaluation of nucleic acid-introducing ability into cell of the peptide (5)

The peptide of SEQ ID NO: 16 was evaluated for its ability of introducing the nucleic acid into various cancer cell lines by the procedure described in Example 9.

The cancer cell used were human uterine cancer cell, MES-SA/Dx5 (purchased from ATCC), a transformant cell from human kidney 293 (purchased from ATCC), human hepatoma cell, SK-HEP-1 (purchased from ATCC), human ovarian cancer cell, SK-OV-3 (purchased from ATCC), rat brain tumor cell, F98 (purchased from ATCC), mouse melanoma cell, B16/BL6 (provided by Chemistry Division, Institute of Immunological Science, Hokkaido University), human uterine cancer cell, MES-SA (purchased from ATCC), mouse lung cancer cell, Lewis Lung Carcinoma (provided by Japanese Foundation for Cancer Research), mouse hapatoma cell, MH134 (provided by Central Laboratories for Experimental Animals), human uterine cancer cell, MES-SA/Mx2 (purchased from ATCC), human medulloblastoma cell, Daoy (purchased from ATCC), human uterine cervix cancer cell, HeLa (purchased from ATCC), human breast cancer cell, MCF7 (purchased from ATCC), human glioblastoma cell, U-87 MG (purchased from ATCC), human breast cancer cell, MDA-MB-468 (purchased from ATCC), human renal cancer cell, A-498 (purchased from ATCC), mouse melanoma cell, B16/F10 (purchased from ATCC), human prostatic cancer cell, DU 145 (purchased from ATCC), human brain tumor cell, U-138 MG (purchased from ATCC) and mouse sarcoma cell, Meth-A (provided by National Cancer Center). The culture media used for the propagation of the cells are indicated in Table 5. It is to be noted that, of the additives, NEAA (manufactured by ICN) is a nonessential amino acid, Na·Pyr (manufactured by ICN) is sodium pyruvate. The FBS used were the one manufactured by NICHIREI in all cases, and the culture media were those manufactured by Life Technologies Oriental. All propagation media were supplemented with penicillin/streptomycin (manufactured by ICN).

Subcultured cells were inoculated on 24 well culture plate at a rate of 1 x 10⁵ cells/well, and the cells were cultivated in the respective culture medium in 5% CO₂ atmosphere at 37°C for 24 hours. After removing the medium, opti-MEM prepared such that the luciferase-expressing plasmid concentration prepared in Example 7 was 1 µg/mL and the peptide concentration prepared in Example 1 was 2.5 µM was added, and the cultivation was continued for 5 hours. The medium was then replaced with the respective propagation medium, and the cultivation was continued in 5% CO₂ atmosphere at 37°C for another 24 hours. Luciferase activity expressed in each cell was then measured by the procedure described in Example 9, and the gene introduction ability of the peptides is indicated in Table 3 in accordance with the criteria described in Example 12.

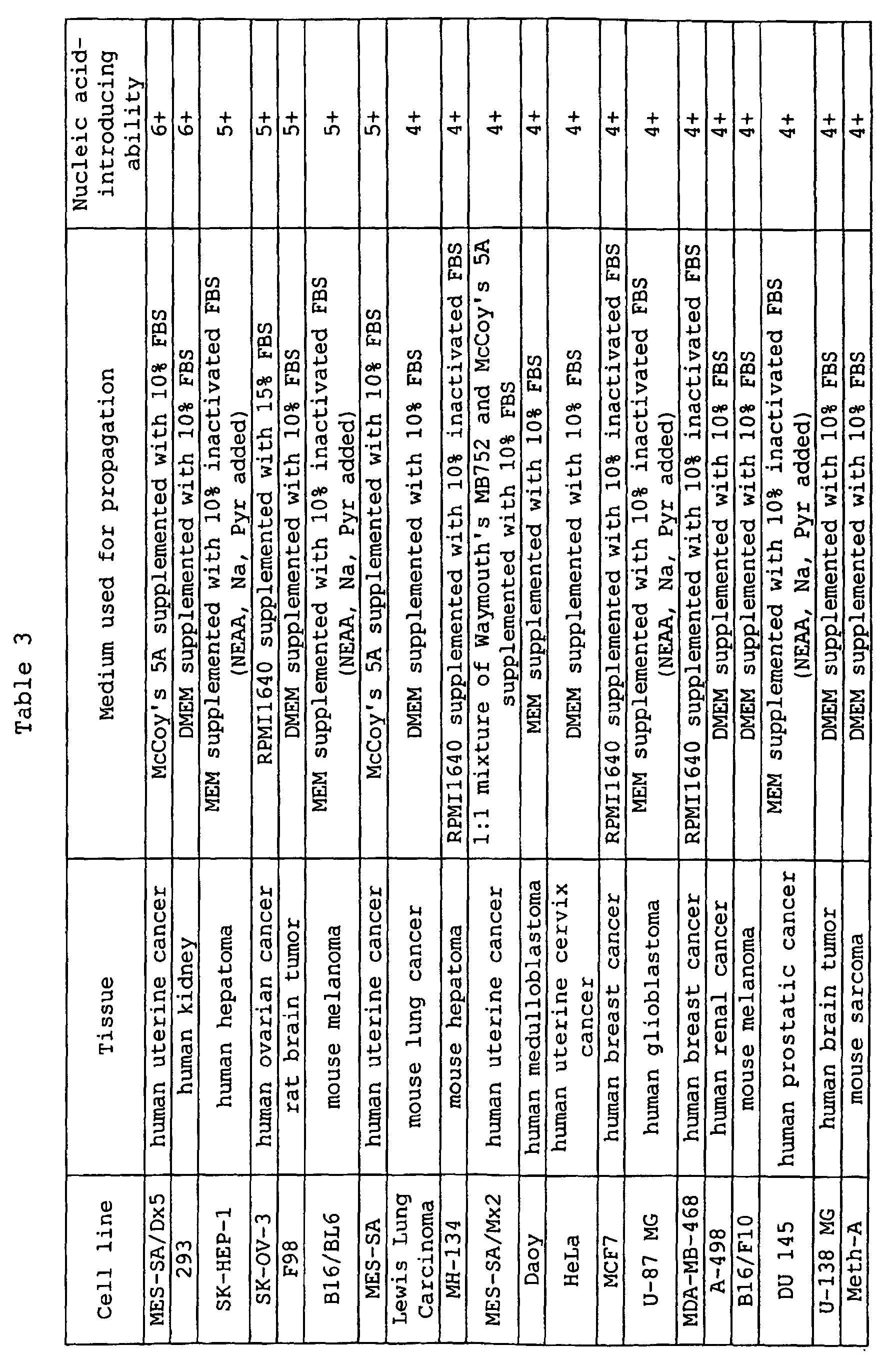

### <Example 16> Evaluation of nucleic acid-introducing ability into cell of the peptide (6)

The HSV-tk-expressing plasmid prepared in Example 7 was introduced in Lewis lung carcinoma cell (mouse lung cancer cell) by using the peptide of SEQ ID NO: 16 prepared in Example 1 to thereby evaluate the effect of increasing the sensitivity for ganciclovir (hereinafter referred to as GCV). To be more specific, cells were inoculated in 96 well culture plate (manufactured by NALGEN NUNC) at a rate of 5 x 10³ cells/well, and cultivated in DMEM supplemented with 10% FBS in 5% CO₂ atmosphere at 37°C for 24 hours. After removing the culture medium, 100 µL of opti-MEM prepared to have a concentration of the HSV-tk-expressing plasmid of 1 µg/mL and a concentration of the peptide of SEQ ID NO: 16 of 2.5 µM was added to each well, and the cultivation was continued for 5 hours. Next, 100 µL of the DMEM supplemented with 20% FBS respectively containing 0, 2, 20, and 200 µM of GCV (Denosine, manufactured by Tanabe Seiyaku) was added to each well, and cultivation was continued in 5% CO₂ atmosphere at 37°C for 3 days. The effect of suppressing the cell propagation was measured by WST-1 assay by adding 10 µL of WST-1 solution (manufactured by Takara Shuzo) in each well, allowing the reaction to proceed for 1 hour, and measuring the absorbance at a wavelength of 620 nm with a multi label counter by using a reference wavelength of 450 nm. As shown in FIG. 13, the cell propagation was found to be suppressed in the case of the Lewis lung carcinoma cell having the HSV-tk-expressing plasmid introduced therein in a manner dependent on the dose of the GCV whereas the effect of increasing the GCV sensitivity was not found in the control cell having the luciferase-expressing plasmid introduced therein.

### <Example 17> Evaluation of storage stability of the complex with nucleic acid

Storage stability of the complex of the plasmid and the peptide was evaluated.

Luciferase-expressing plasmid was introduced in Vero cell in accordance with the procedure described in Example 9 by using the opti-MEM prepared to have a concentration of the luciferase-expressing plasmid prepared in Example 7 of 2 µg/mL and a concentration of the peptide of SEQ ID NO: 4 prepared in Example 1 of 5 µM to thereby measure the luciferase activity. The opti-MEM containing plasmid and peptide used were the one which had been prepared immediately before the gene introduction, and the one which had been prepared 1 week (7 days) in advance and stored at 4°C.

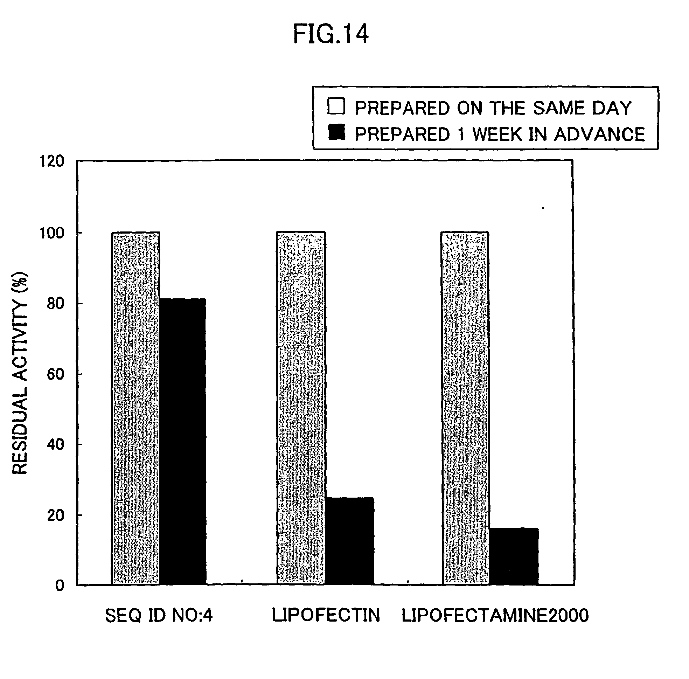

FIG. 14 shows gene-introducing activity of the peptide/plasmid complex stored at 4°C for 1 week as a value in relation to the gene-introducing activity of the peptide/plasmid complex prepared immediately before the gene introduction which is assumed to be 100%. As shown in FIG. 14, the gene-introducing activity of the peptide/plasmid complex stored at 4°C for 1 week was substantially equivalent to that of the gene-introducing activity of the peptide/plasmid complex prepared immediately before the gene introduction.

For the purpose of reference, gene-introducing activity of Lipofectin and LipofectAMINE 2000 (manufactured by Life Technologies Oriental), which are commercially available plasmid-introducing agents, were also evaluated by mixing these reagent with luciferase-expressing plasmid in accordance with the attached protocol, and evaluating their gene-introducing activity after storing at 4°C for 1 week. Both Lipofectin and LipofectAMINE 2000 exhibited a marked decrease in the gene-introducing activity. The results are also shown in FIG. 14.

### <Example 18> Evaluation of nuclease resistance-imparting ability (1)

Nuclease resistance-imparting ability for natural (P=O form) oligonucleotide was evaluated. To be more specific, 2 U of nuclease Bal 31 (manufactured by Takara Shuzo) was added to 20 mM Tris-HCl buffer (pH = 8) containing 50 µM of the peptide of SEQ ID NO: 1 prepared in Example 1, 3 µM of P=O form oligonucleotide prepared in Example 6, 150 mM NaCl, 12 mM MgCl₂, and 12 mM CaC₁₂, and the reaction was allowed to take place at 30°C for 60 minutes. Next, the oligonucleotide which escaped from the decomposition was extracted with phenol/chloroform, and subjected to electrophoresis by the procedure described in Example 8 to stain the oligonucleotide.

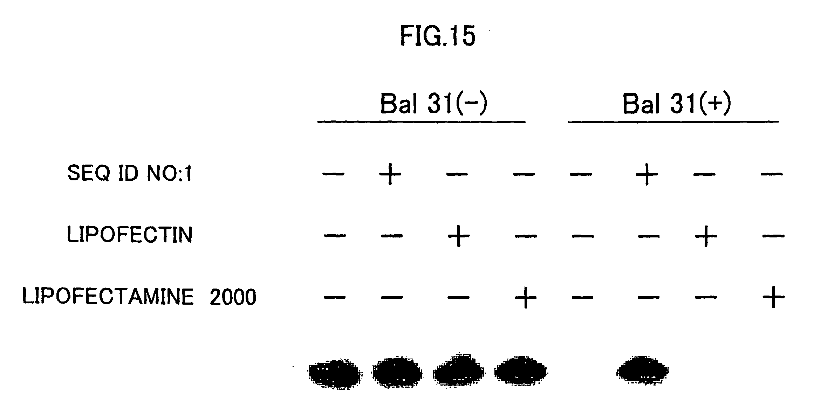

FIG. 15 shows the electropherogram. As shown in FIG. 15, the peptide of SEQ ID NO: 1 was found to exhibit nuclease resistance-imparting ability for the natural (P=O form) oligonucleotide.

For the purpose of reference, nuclease resistance-imparting ability was also evaluated for lipofectin and LipofectAMINE 2000, which are commercially available plasmid-introducing agents. Both lipofectin and LipofectAMINE 2000 exhibited no stained oligonucleotide in the electropherogram, indicating the decomposition of the oligonucleotide. The results are shown in FIG. 15.

### <Example 19> Evaluation of nuclease resistance-imparting ability (2)

pBR322 prepared by the procedure described in Example 7 (final concentration, 20 µg/mL) and the peptide of SEQ ID NO: 16 prepared in Example 1 were mixed in 20 mM Tris-HCl buffer (pH = 8) containing 150 mM NaCl and 1.7 mM MgCl₂ at a charge ratio (+/- ratio) of 0 to 10, and 5 U of DNase I (manufactured by Takara Shuzo) was then added. After allowing the reaction to proceed at 30°C for 60 minutes, the plasmid which escaped the decomposition was extracted with phenol/chloroform, and subjected to electrophoresis by the procedure described in Example 8 to stain the plasmid.

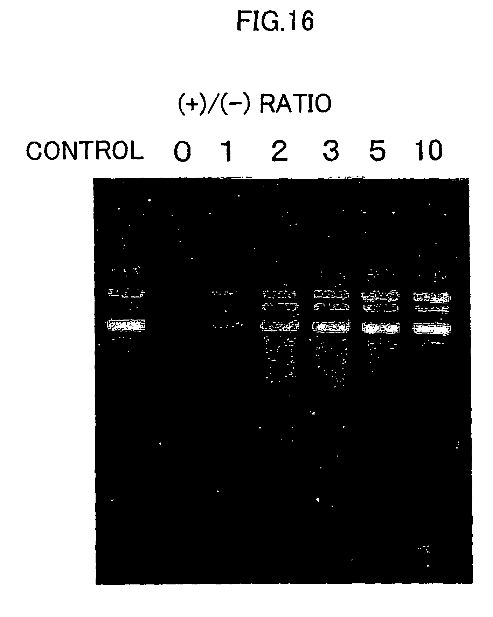

FIG. 16 shows the electropherogram. As shown in FIG. 16, the peptide of SEQ ID NO: 16 was found to exhibit nuclease resistance-imparting ability for the plasmid.

### <Example 20> Evaluation of the affinity of the peptide for phosphatidyl serine (EIA)

The specific affinity of the peptide for phosphatidyl serine was evaluated by measuring activity of the peptide for inhibiting the binding of the human Factor VIII to phosphatidyl serine by using human Factor VIII which is known to bind to phosphatidyl serine but not to phosphatidyl choline in the presence of serum albumin. To be more specific, 100 µL of ethanol solution containing 10 µg/mL of phospholipids at a phosphatidyl serine (manufactured by SIGMA): phosphatidyl choline (manufactured by SIGMA) ratio of 3:7 was added to the wells of a 96 well plate (Immulon I, manufactured by Dynatech), and exsiccated by using a centrifugal evaporator (EC-95C, manufactured by Sakuma Seisakusho) at 40°C for 40 minutes. 200 µL each of TBS (10 mM Tris-HCl (pH 7.4) containing 1% bovine serum albumin (BSA Fraction V, hereinafter referred to as BSA, manufactured by Seikagaku Corporation) and 0.9% (W/V) NaCl solution were added to each well, and the wells were blocked by allowing the plate to stand at 37°C for 2.5 hours. After washing the plate with water, 100 µL of TBS solution supplemented with 5% BSA mixed with human Factor VIII (manufactured by American Diagnostica) at a final concentration 1 µg/mL and the peptide of predetermined dose prepared in Example 1 was added to each well, and the reaction was allowed to take place at 4°C for 24 hours.

After the completion of the reaction, the human Factor VIII that became bound to the phosphatidyl serine was measured by enzyme immunoassay (EIA) in accordance with the general procedure described in a book ("Enzyme Immunoassay (3rd ed.)", Eiji Ishikawa et al., Igaku-Shoin, 1987). To be more specific, the measurement was conducted by using anti-human Factor VIII mouse monoclonal antibody (ESH8, manufactured by American Diagnostica) for the primary antibody, horseradish peroxidase-labeled anti-mouse IgG antibody (P0260, manufactured by Daco) for the secondary antibody, and tetramethylbenzidine for the chromogenic substrate, and measuring the absorption at a wavelength of 450 nm by using a reference wavelength of 630 nm on a spectrophotometer (NJ-2100, manufactured by Intermed).

Intensity of the affinity of the peptide for the phosphatidyl serine was evaluated by using the value obtained by subtracting the absorption of the well having no human Factor VIII added thereto from the absorption of the well having only the human Factor VIII added thereto as the control value (100%), and designating the peptide concentration at which the value 0.5 was obtained when the value obtained by subtracting the absorption of the well having only the peptide added thereto from the absorption of the well having a mixture of the human Factor VIII and the peptide added thereto were divided by the control value as the IC₅₀ value, and evaluating the intensity to be ++ when the IC₅₀ value was up to 1 µM, and + when the intensity was more than 1 µM but not more than 10 µM. It is to be noted that the specific affinity was evaluated to be none when the intensity was 10 µM or more. The results are shown in Table 4.

**Table 4**

| SEQ ID NO: | Affinity for phosphatidyl serine |
|---|---|
| SEQ ID NO:1 | ++ |
| SEQ ID NO:2 | ++ |
| SEQ ID NO:3 | ++ |
| SEQ ID NO:5 | ++ |
| SEQ ID NO:6 | ++ |
| SEQ ID NO:7 | + |
| SEQ ID NO:8 | + |
| SEQ ID NO:10 | + |
| SEQ ID NO:11 | + |
| SEQ ID NO:15 | ++ |
| SEQ ID NO:16 | ++ |
| SEQ ID NO:17 | ++ |
| SEQ ID NO:19 | ++ |
| SEQ ID NO:23 | ++ |

### <Example 21> Evaluation of the affinity of the peptide for phosphatidyl serine (2)

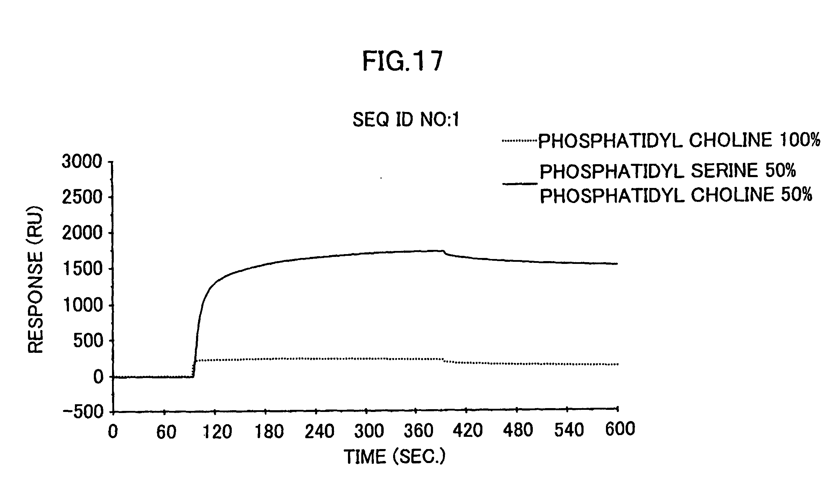

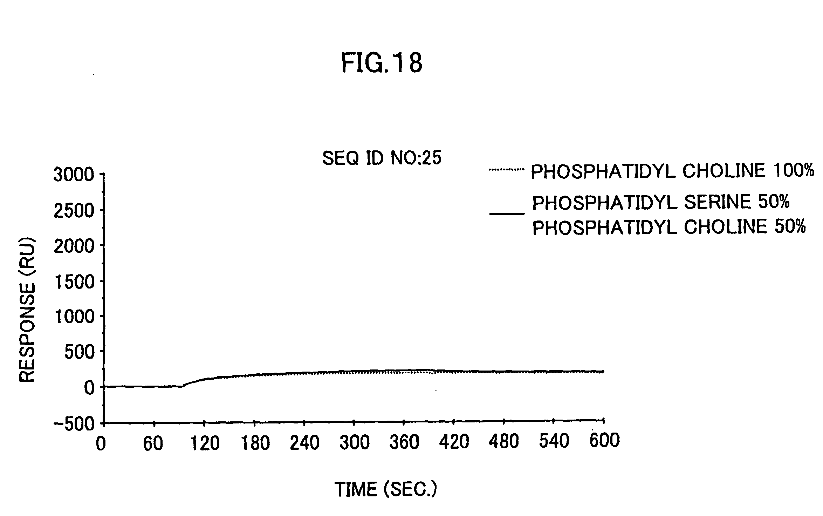

The specific affinity of the peptide for phosphatidyl serine was evaluated by means of surface plasmon resonance (SPR) using Biacore 2000 (manufactured by Biacore). To be more specific, phosphatidyl choline was immobilized on flow cell 1, and 50% of phosphatidyl serine and 50% of phosphatidyl choline were immobilized on flow cell 2 of HPA chip (manufactured by Biacore), and ability of the peptide to bind to the phospholipid was evaluated in the presence of 0.1 mg/mL BSA to thereby evaluate the affinity. It was then found that the binding of the peptide SEQ ID NO: 1 to the flow cell 2 having 50% of phosphatidyl serine and 50% of phosphatidyl choline immobilized thereto was stronger than that of the flow cell 1 having phosphatidyl choline immobilized thereto, and the affinity of the peptide specific for the phosphatidyl serine was thereby indicated (FIG. 17). In contrast, the peptide of SEQ ID NO: 25 wherein the sequence of SEQ ID NO: 1 had been randomized showed no binding to neither the flow cell 1 having phosphatidyl choline immobilized thereto nor the flow cell 2 having 50% of phosphatidyl serine and 50% of phosphatidyl choline immobilized thereto, indicating the absence of the affinity (FIG. 18).

### <Example 22> Correlation between the amount of phosphatidyl serine translocation and the introduction efficiency (selectivity)

In order to elucidate the correlation between the level of the gene-introducing ability of the peptide and the affinity for phosphatidyl serine, an investigation was conducted by using cells exhibiting different amount of phosphatidyl serine translocation to the cell surface. To be more specific, the cells used were Vero, A549, and T24 cells, and the luciferase-expressing plasmid prepared in Example 7 was introduced in the cells by using the peptide of SEQ ID NO: 1 prepared in Example 1 in accordance with the description of Example 9 to thereby measure luciferase activity. In the meanwhile, the amount of phosphatidyl serine translocated to the outer surface of the cell membrane of the cells was measured by labeling the cells with FITC-labeled ANNEXIN V (ANNEXIN V-FITC, manufactured by Pharmingen) in accordance with the manual attached thereto, and conducting flow cytometry (FACS Calibur, manufactured by Becton Dickinson). To be more specific, the cells that had been scraped from the culture flask were mixed with the solution of the FITC-labeled ANNEXIN V, and the cells were measured for their FITC fluorescent intensity by flow cytometry. Average fluorescent intensity of each cell was then designated the amount of phosphatidyl serine translocation for each cell line.

Correlation was then found between the amount of phosphatidyl serine translocation (amount of ANNEXIN V binding) and the gene introduction activity of the peptide vector as shown in Table 5.

For the purpose of reference, the procedure as described above was repeated by using lipofectin (manufactured by Life Technologies Oriental) which is a commercially available plasmid-introducing agent in accordance with the attached protocol. It was then found that the plasmid was equally introduced in every type of cells, indicating the absence of the specific recognition of the phosphatidyl serine.

**Table 5**

| | | Luciferase activity (cps/mg protein) | |
|---|---|---|---|
| Cell | Amount of Annexin-V bound | Peptide (SEQ ID NO: 1) | Lipofectin |
| Vero | 165 | 2.5 x 10⁶ | 1.1 x 10⁶ |
| A549 | 82 | 8.7 x 10⁴ | 8.4 x 10⁵ |
| T24 | 32 | 7.8 x 10³ | 1.1 x 10⁶ |

### <Example 23> Correlation between the amount of phosphatidyl serine translocation and the introduction efficiency (2)

In order to elucidate the correlation between the gene-introducing ability of the peptide and the affinity for the phosphatidyl serine, nucleic acid-introducing ability of the peptide was investigated by using the cells wherein the phosphatidyl serine had not been translocated, and the cells wherein the phosphatidyl serine has been translocated by stimulating the cell. The cell used was RBL-2H3 cell from rat basophil (purchased from ATCC), and this cell was sensitized with anti-DNP mouse monoclonal IgE antibody (manufactured by SIGMA) and degranulated with DNP-BSA (manufactured by Calbiochem). Luciferase gene was introduced to such cell by using the peptide of SEQ ID NO: 16 in accordance with the procedure described in Example 9 to thereby measure the luciferase activity. To be more specific, RBL-2H3 cells were inoculated in a 24 well plate at a rate of 3 x 10⁵ cells/well, and after adding anti-DNP mouse monoclonal IgE antibody to a concentration of 100 ng/mL, the cells were cultivated for 24 hours. After washing the cells twice with PBS, DNP-BSA was added to 10 ng/mL to cause degranulation for 45 minutes. After removing the culture medium and washing the well with physiological saline, opti-MEM having a concentration of the luciferase-expressing plasmid prepared in Example 7 of 1 µg/mL and a concentration of the peptide of SEQ ID NO: 16 prepared in Example 1 of 2.5 µM was added, and cultivation was continued for 5 hours.

The medium was then replaced with MEM supplemented with 15% inactivated FBS (having NEAA and Na·Pyr added thereto), and incubation was continued in 5% CO₂ atmosphere at 37°C for 1 day. The cells were then scraped off, and evaluated for their luciferase activity by the procedure described in Example 9. In the meanwhile, amount of phosphatidyl serine translocated in the RBL-2H3 cell by degranulation was measured by the procedure as described below, namely, by inoculating the RBL-2H3 cell in a 6 well culture plate (manufactured by NALGEN NUNC) at a rate of 1.5 x 10⁶ cells per well, adding anti-DNP mouse monoclonal IgE antibody to a concentration of 100 ng/mL, and cultivating in MEM supplemented with 15% inactivated FBS (having NEAA and Na·Pyr added thereto) in 5% CO₂ atmosphere at 37°C for 24 hours. After washing the wells twice with PBS, DNP-BSA was added to 10 ng/mL to thereby cause degranulation for 45 minutes. After scraping off the cells, the cells were labeled with FITC-labeled ANNEXIN V (manufactured by MBL) in accordance with the procedure described in the attached manual, and the activity was measured by flow cytometry.

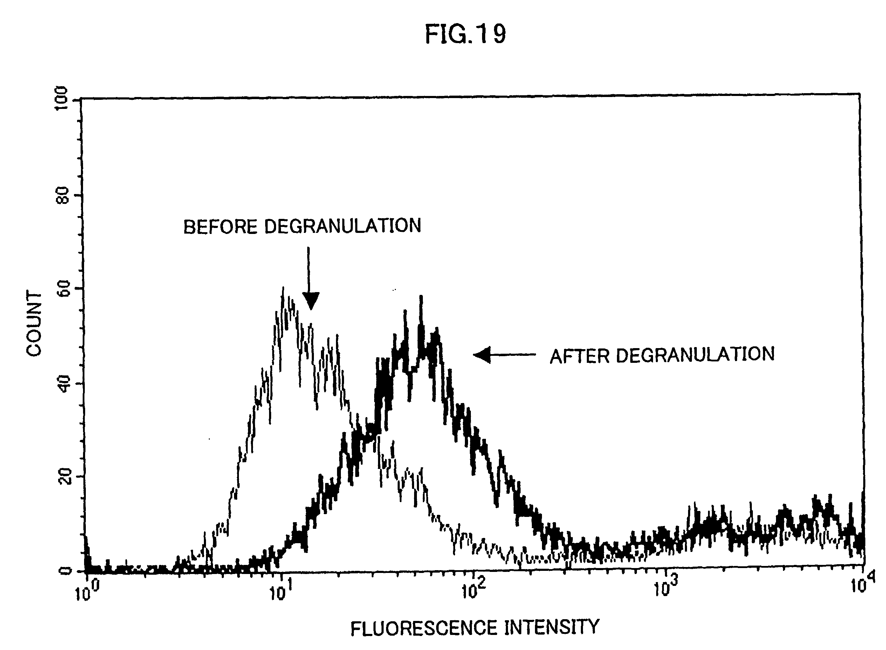

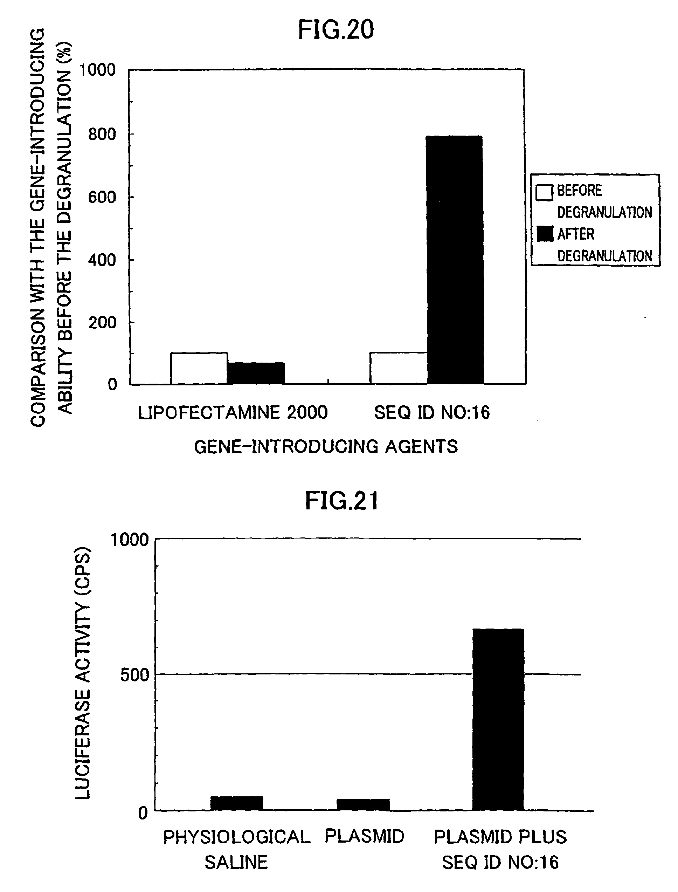

It was then found that the cells stimulated for degranulation had the phosphatidyl serine translocated to its surface (FIG. 19), and the gene-introducing ability of the peptide into the RBL-2H3 cell that had been stimulated for degranulation was significantly high compared to the case of the undegranulated cell (FIG. 20).

It is to be noted that the gene-introducing ability of LipofectAMINE 2000 which is a commercially available gene-introducing agent was equivalent or slightly lower in the case of the degranulated cell compared to the case of the cell before the degranulation.

### <Example 24> Evaluation of nucleic acid-introducing ability into cell of the peptide (7)

In order to demonstrate the in vivo gene-introducing ability of the peptide, nucleic acid-introducing ability of the peptide was examined by using an ascites cancer model animal having Meth-A mouse sarcoma cells transplanted thereto. To be more specific, 4 x 10⁶ Meth-A cells were transplanted in the abdominal cavity of BALB/c mouse (purchased from Charles River JAPAN), and after 4 days, a mixture of 30 µg of the luciferase-expressing plasmid prepared in Example 7 and 113 nmol of the peptide of SEQ ID NO: 16 prepared in Example 1 was administered to the abdominal cavity of the animal. The mouse was killed after another 1 day, and Meth-A cell in the abdominal cavity was recovered to thereby measure the luciferase activity.

It was then found that, as shown in FIG. 21, while no expression of luciferase was found in the contrast mouse which had been administered solely with physiological saline or in the contrast mouse which had been administered only with 30 µg luciferase-expressing plasmid, luciferase was found to be expressed in the mouse which had been administered with a mixture of 30 µg of the luciferase-expressing plasmid and 113 nmol of the peptide of SEQ ID NO: 16 to its abdominal cavity.

### <Example 25> Evaluation of nucleic acid-introducing ability into cell of the peptide (8)

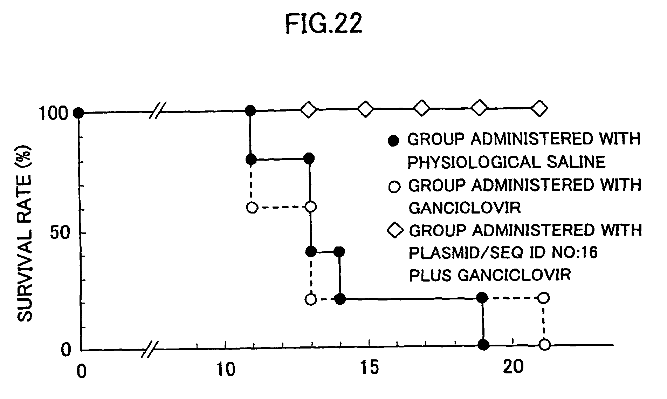

In order to demonstrate the pharmacological effects of the in vivo gene introduction by using the peptide, anti tumor action realized by the increase of GCV sensitivity by the introduction of HSV-tk gene was examined by using the model animal having Lewis lung carcinoma cell (mouse lung cancer cell) inoculated in its abdominal cavity. To be more specific, 1 x 10⁵ Lewis lung carcinoma cells were transplanted in the abdominal cavity of a C57BL/6 mouse (purchased from Charles River JAPAN), and a mixture of 10 µg of the HSV-tk-expressing plasmid prepared in Example 7 and 40 nmol of the peptide of SEQ ID NO: 16 prepared in Example 1 was administered in the abdominal cavity after the cell transplantation. The mouse was also administered with GCV at a dose of 30 mg/kg/day from 1st to 8th day after the transplantation. As shown in FIG. 22, it was then found that while the average survival period of the mice was 14 days both in the case of the group administered with the physiological saline and in the case of the group administered only with GCV at a dose of 30 mg/kg/day, all mice were alive 20 days after the cell transplantation in the case of the group administered with the mixture of 10 µg of the HSV-tk-expressing plasmid and 40 nmol of the peptide of SEQ ID NO: 16 followed by the administration of the GCV at a dose of 30 mg/kg/day.

### <Example 26> Evaluation of toxicity of the peptide

The peptide used in the present invention was administered to a mouse from its tail vein to thereby evaluate its toxicity. The mouse used was a female BALB/c mouse of 5 week old, and the peptide was used at a dose of 5 mg/kg. The peptide (SEQ ID NO: 16) used in the present invention was found to induce no significant effects in the mouse, demonstrating that no aggregates were formed in the blood by the peptide of the present invention, and that the peptide of the present invention is highly safe.

### <Example 27> Modification with PEG of the plasmid /peptide complex

A complex of a plasmid and the peptide was modified with activated polyethylene glycol (PEG).

First, the PEG-modified plasmid / peptide complex for use in the in vitro gene introduction was prepared by the procedure as described below.

100 µL of physiological saline containing a plasmid including luciferase gene (pCMV-Luc) at a concentration of 16 µg/mL and 100 µL of physiological saline containing the peptide of SEQ ID NO: 16 at a concentration of 48 µM were preliminarily mixed at room temperature.

To this mixture was added 10 µL of physiological saline containing mPEG-SPA5000 (succinimidyl ester of methoxy poly(ethylene glycol)propionic acid, Average M.W. 5000, manufactured by Shearwater) at a concentration of 23 mg/mL, and the reaction was allowed to proceed at room temperature for 1 hour to produce the PEG-modified pCMV-Luc/ peptide complex (PEG1).

Next, PEG-modified plasmid / peptide complex for use in the in vivo gene introduction was prepared by the procedure as described below.

1050 µL of 5% glucose solution containing the plasmid (pCMV-Luc) as described above at a concentration of 268 µg/mL and 1050 µL of 5% glucose solution containing the peptide of SEQ ID NO: 16 at a concentration of 804 µM was preliminarily mixed at room temperature to form a complex.

To this mixture was added 150 µL of 5% glucose solution containing the mPEG-SPA5000 as described above at a concentration of 263.8 mg/mL, and reaction was allowed to proceed at room temperature for 1 hour to obtain PEG-modified pCMV-Luc/ peptide complex (PEG2).

### <Example 28> Evaluation of gene introduction ability of the PEG-modified plasmid / peptide complex (in vitro)

The pCMV-Luc/ peptide complex (PEG1) produced by the procedure described in Example 27 or the complex of pCMV-Luc and the peptide of SEQ ID NO: 16 (PEG-undmodified complex) was introduced in Vero cell at a plasmid concentration of 1 µg/mL by the procedure described in Example 9 to measure the luciferase activity expressed.

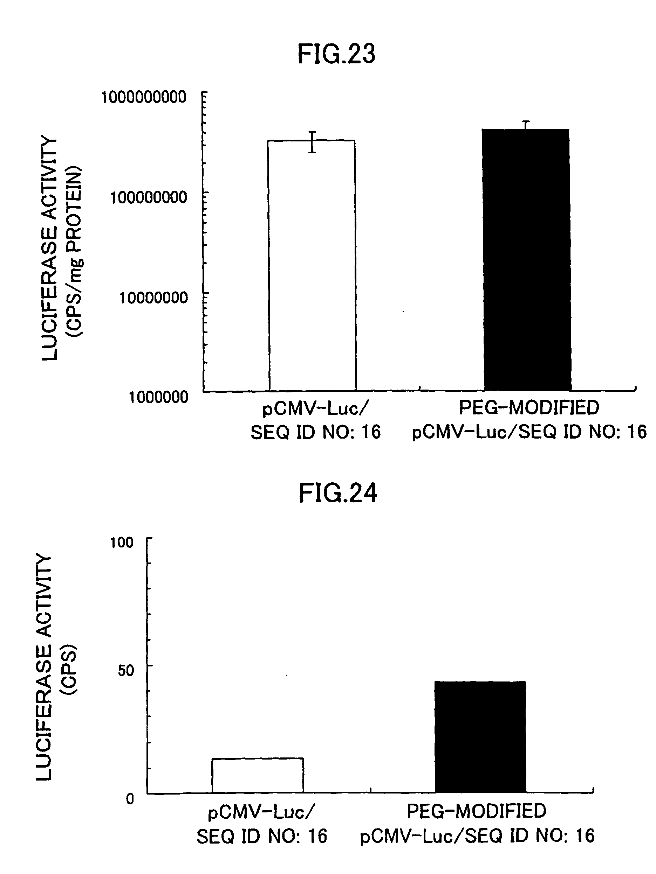

As demonstrated by the results shown in FIG. 23, the luciferase activity of the Vero cell having the PEG-modified complex introduced was equivalent to the luciferase activity of the Vero cell having the PEG-unmodified complex introduced, confirming that the gene introduction ability of the plasmid / peptide complex is not reduced by the PEG modification.

### <Example 29> Evaluation of gene introduction ability of the PEG-modified plasmid / peptide complex (in vivo)

Gene introduction ability of the PEG-modified plasmid / peptide complex was evaluated in vivo by using anaphylaxis shock mouse.

First, mice were sensitized with ovalbumin (hereinafter referred to as OVA) by the procedure as described below to obtain anaphylaxis shock mice. OVA (Egg Alubumin, 5x Cryst, manufactured by Seikagaku Corporation) was first adjusted to 32 µg/mL by using 1.8% solution of sodium chloride.

Next, aluminum hydroxide gel (Alu-Gel-S, manufactured by SERVA) was adjusted to 8 mg/mL with ultrapure water, and this solution was cooled on ice. To this solution was mixed an equal volume of the OVA solution as described above with stirring to prepare OVA/aluminum hydroxide gel solution.

500 µL of this OVA/aluminum hydroxide gel solution was then administered to abdominal cavity of BALB/c mouse (male, 4 week old, Japan Charles River) for sensitization with the OVA. The sensitization was conducted twice at an interval of 5 days.

The gene introduction was conducted on the 13th day after the start of the sensitization.

To be more specific, the PEG-modified pCMV-Luc/peptide complex (PEG2) produced by the procedure described in Example 27 or the (PEG-undmodified) complex of pCMV-Luc and the peptide of SEQ ID NO: 16 was systemically administered to the mice that had been sensitized with OVA as described above from their tail vein at a dose of 50 µg calculated in terms of pCMV-Luc simultaneously with 50 µg of OVA.

Right lung was extirpated the next day, and the extirpated lung was homogenized in 500 µL of Lysis Buffer (manufactured by Promega) using Handy Pestle (manufactured by Toyobo). The lysate was centrifuged by a small-size centrifuge (manufactured by Tomy) at 12000 rpm for 15 minutes, and luciferase activity was measured for 20 µL of the supernatant by the procedure of Example 9.

As demonstrated in the results shown in FIG. 24, the luciferase activity was clearly higher in the lung of the mouse administered with the PEG-modified complex compared to that in the lung of the mouse administered with the corresponding PEG-unmodified complex.

### <Example 30> Identification of PEG-modified peptide in the PEG-modified plasmid / peptide complex

550 µL of the reaction solution of the pCMV-Luc /peptide complex (PEG1) prepared in Example 27 was charged in a centrifugal ultrafiltration device (CentriconYM-100, manufactured by Amicon), and centrifuged at 4°C and 1,000 xg for 10 minutes to separate and remove the free peptide and the free PEG which had not reacted, and the step of adding 500 µL of physiological saline and centrifuging at 4°C and 1,000 xg for 10 minutes was repeated for another 3 times. After washing, 50 µL of non-reduced SDS sample buffer (manufactured by Daiichi Pure Chemicals) was added, and the reaction system was allowed to stand at room temperature for 5 minutes to collect the sample solution (M) containing pCMV-Luc / peptide complex (PEG1) remaining on the ultrafiltration membrane.

Next, 25 µL of this sample solution (M) was subjected to SDS polyacrylamide gel electrophoresis (constant current of 20 mA, 90 minutes) by using 5 to 20% gradient gel (manufactured by Atto). The gel after the electrophoresis was stained by using Silver Staining Kit II (manufactured by WakoPure Chemicals).

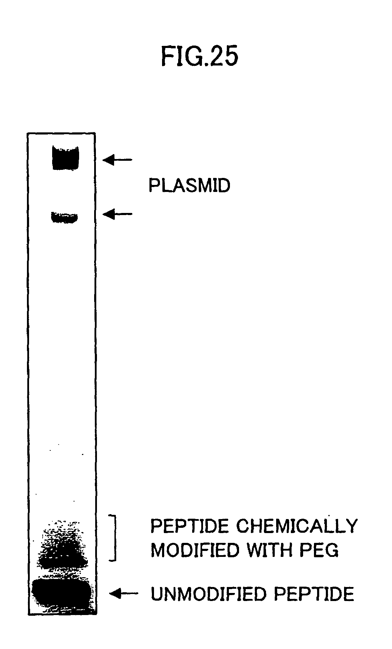

As a consequence, a broad stained image was found in the vicinity of the molecular weight of 10,000 Da to 20,000 Da as shown in FIG. 25, and since the molecular weight of the peptide and the molecular weight of the PEG per 1 molecule are about 5,000 Da, respectively, the number of the PEG molecules bonded to 1 peptide molecule was deduced to be 1 to 3.

### <Example 31> Chemical synthesis and confirmation of the peptide used for modification

Peptides having cysteine attached to the C terminal or N terminal of the peptide of SEQ ID NO: 16 (hereinafter referred to as peptide 16CC and peptide 16NC respectively) were synthesized by solid phase synthesis by using an automatic peptide synthesizer (model 433 manufactured by Applied Biosystems) in accordance with the manufacturer's manual. The peptide was cleaved, deprotected, precipitated in ether, stripped of the ether, dissolved in distilled water, and lyophilized. Next, the peptide was dissolved in 20% acetonitrile aqueous solution containing 10mM HCl. By using C18 column (CAPCELLPAK C18AG120, manufactured by Shiseido) and high performance liquid chromatography (625 LC System, manufactured by Waters), the peptide was obtained in linear concentration gradient of 20% to 70% acetonitrile aqueous solution containing 10 mM HCl. The thus purified peptides were lyophilized, dissolved in distilled water, and stored after lyophilization. The yield was 40 mg to 50 mg, respectively.

Next, the thus obtained peptide was confirmed that it was the peptide desired by the procedure described in Example 2, and the peptide concentration was determined by the procedure described in Example 3.

### <Example 32> Preparation of the peptide chemically modified with PEG having a phospholipid bonded thereto

"Peptides chemically modified with PEG having a phospholipid bonded thereto" were prepared by bonding peptide 16CC or peptide 16NC on the terminal of the PEG moiety having maleimide group on its end and synthetic phospholipid added thereto (DSPE-20MA, manufactured by NOF corporation) (hereinafter referred to as DSPE-16CC and DSPE-16NC, respectively). More specifically, an aqueous solution of DSPE-20MA at 6 µmol/mL was prepared, and aqueous solutions of peptide 16CC and aqueous solutions of peptide 16NC of 150 nmol/mL, 300 nmol/mL, and 600 nmol/mL were prepared. Next, 30 µL of DSPE-20MA solution and 30 µL of peptide solutions of different concentration were mixed in equal amount, and the mixtures were allowed to react at room temperature for 2 hours to promote conversion of 2. 5%, 5%, and 10% of the DSPE-20MA to DSPE-16CC or DSPE-16NC. The reaction was then terminated by adding 30 µL of 18 µmol/mL aqueous solution of cysteine. A control was prepared by adding 30 µL of water instead of the peptide solution.

### <Example 33> Preparation of doxorubicin-containing liposome modified with the peptide chemically modified with PEG

A liposome preparation modified with the peptide chemically modified with PEG was prepared. First, a doxorubicin-containing liposome was produced using negatively charged liposome (EL-A-01, manufactured by NOF corporation) and doxorubicin hydrochloride (manufactured by Wako Pure Chemical) by suspending EL-A-01 in 5% glucose solution at 50 µmol/mL and suspending doxorubicin hydrochloride in 5% glucose solution at 5 µmol/mL, mixing 30 µL of the EL-A-01 suspension and 30 µL of the doxorubicin hydrochloride solution at equal amount, and heating the mixture to 60°C for 5 minutes. The DSPE-16CC or the DSPE-16NC prepared in Example 32 was fused to the surface of the thus produced doxorubicin-containing liposome by mixing the solution of the doxorubicin-containing liposome (60 µL) with 75 µL of the DSPE-16CC solution or the DSPE-16NC solution prepared in Example 32, and heating the mixture to 60°C for 5 minutes.

The reaction mixture was centrifuged (10,000 xg, 20 minutes, 4°C), and the supernatant was removed to remove the doxorubicin that had not been incorporated in the liposome. After repeating this procedure once, the centrifugate was suspended in 120 µL of 5% glucose solution to obtain doxorubicin-containing liposome modified with the peptide chemically modified with PEG.

### <Example 34> Quantitation of doxorubicin in the doxorubicin-containing liposome modified with the peptide chemically modified with PEG

Concentration of the doxorubicin in the liposome prepared in Example 33 was quantitatively measured by mixing 20 µL of the solution of the liposome prepared in Example 33 and 180 µL of 5% glucose solution, diluting 10 times, further adding 400 µL of isopropyl alcohol to the mixture to lyse the liposome, and measuring absorption of the solution at 470 nm to determine the amount of doxorubicin released. In the meanwhile, calibration curve was depicted by preparing 5% glucose solutions containing 0, 12.5, 25, 50, and 100 µg/mL of doxorubicin for use as standard samples, and mixing 200 ml of this solution with 400 µL of the isopropyl alcohol.

### <Example 35> Evaluation of the doxorubicin-containing liposome modified with the peptide chemically modified with PEG for its effect on suppressing the cell propagation (1)

The doxorubicin-containing liposome modified with DSPE-16CC produced by the procedure of Example 33 was evaluated for its effect of suppressing the cell propagation using B16-BL6 mouse melanoma cell and Meth-A mouse sarcoma cell.

First, amount of the phosphatidyl serine on the cell surface was measured by the procedure described in Example 23. When the B16-BL6 cell and the Meth-A cell were treated with FITC-labeled Annexin V and measured by flow cytometry, average fluorescence intensity of the B16-BL6 was 735 while the average fluorescence intensity of Meth-A cell was 114, and the average fluorescence intensity was higher in the B16-BL6 cell.

Next, the doxorubicin-containing liposome modified with the peptide chemically modified with PEG was evaluated for its effect on suppressing the cell propagation by inoculating the B16-BL6 cell and the Meth-A cell in 96 well plate at 1×10³ cells per well, respectively, and incubating the cells at 37°C in 5% CO₂ atmosphere for 1 day. On the next day, the doxorubicin-containing liposome modified with the DSPE-16CC was added to each cell at a dose in concentration calculated in terms of doxorubicin of 0, 10, 30, 100, 300, 1000, and 3000 ng/mL, and the cells were incubated at 37°C in 5% CO₂ atmosphere for 2 days. Ten µL of WST-1 solution (manufactured by TAKARA) was then added to each well for color development. The effect of suppressing the cell propagation was evaluated by comparing the activity of each sample with the absorption (100%) of the group with no addition of the sample. It was then found that the doxorubicin-containing liposome wherein 2. 5%, 5%, or 10% of the entire PEG molecules on the liposome corresponds to the PEG molecules chemically bonded to the peptide had the effect of suppressing the cell propagation expressed by IC50 value which was about 3 times at maximum higher than that of the doxorubicin-containing liposome containing no peptide chemically modified with PEG in the case of Meth-A cell with less expression of phosphatidyl serine, and the effect about 33 times at maximum higher in the case of B16-BL6 cell with higher expression of phosphatidyl serine (Table 6).

**Table 6**

| (PEG having the peptide of SEQ ID NO: 16 bonded thereto / total number of the PEG molecule) x 100 [%] | IC₅₀ [ng/mL] | |
|---|---|---|
| | B16-BL6 | Meth-A |
| 0 | 10000 | 2500 |
| 2.5 | 1500 | 2500 |
| 5 | 300 | 1500 |
| 10 | 350 | 900 |

### <Example 36> Evaluation of the doxorubicin-containing liposome modified with the peptide chemically modified with PEG for its effect on suppressing the cell propagation (2)

The doxorubicin-containing liposome modified with DSPE-16NC prepared by the procedure of Example 33 was evaluated for its effect of suppressing cell propagation using B16-BL6 mouse melanoma cell by the procedure described in Example 35. It was then found that the doxorubicin-containing liposome wherein 2. 5%, 5%, or 10% of the entire PEG molecules on the liposome correspond to the PEG molecules chemically bonded to the peptide had the effect of suppressing the cell propagation expressed by IC₅₀ value which was about 10 times at maximum higher than that of the doxorubicin-containing liposome containing no peptide chemically modified with PEG (Table 7).

**Table 7**

| (PEG having the peptide of SEQ ID NO: 16 bonded thereto / total number of the PEG molecule) x 100 [%] | IC₅₀ [ng/mL] |
|---|---|
| 0 | 2000 |
| 2.5 | 800 |
| 5 | 250 |
| 10 | 200 |

### <Example 37> Effect of the doxorubicin-containing liposome modified with the peptide chemically modified with PEG in extending the survival period of cancer-bearing mice

The doxorubicin-containing liposome modified with DSPE-16NC prepared by the procedure of Example 33 was evaluated for its effect of extending the survival period of a cancer-bearing mice carrying a B16-BL6mouse melanoma cell by intradermally transplanting 1.0×10⁶ B16-BL6 mouse melanoma cells in C57/BL6 mice (7 week old, female, Japan Charles River), and grouping the mice 6 days after the transplantation using the tumor volume for the index. Administration in the case of control (only 5% glucose solution) and the administration of liposome preparation (at a dose of 5 mg/kg calculated in terms of doxorubicin) were carried out after 6 days and 9 days of the cell transplantation. It was then found that the average survival period of the cancer-bearing mouse administered only with 5% glucose was 30.4 days, while the average survival period of the cancer-bearing mouse administered with the doxorubicin-containing liposome containing no peptide chemically modified with PEG was 39.2 days and the cancer-bearing mouse administered with the doxorubicin-containing liposome wherein 6% of the entire PEG molecules on the liposome correspond to the peptide chemically modified with PEG was 48.0 days.

### <Comparative Example 1>

A peptide (SEQ ID NO: 25) having an amino acid composition which is the same as that of the peptide of SEQ ID NO: 1 but with an utterly random amino acid sequence, hence a sequence which does not include "the sequence of 18 amino acids exhibiting the four sided structure of the present invention" was evaluated for its nucleic acid-introducing ability by the procedure described in Example 9. This peptide was also evaluated.for its affinity for phosphatidyl serine by the procedure described in Example 20. The results indicate that this peptide had neither the nucleic acid-introducing ability nor the affinity for phosphatidyl serine.

CD spectrum was also measured by the procedure described in Example 5. The results indicate that no α-helix structure is found even in the presence of SDS.

### <Comparative Example 2>

SEQ ID NO: 26 which was prepared by conducting amino acid substitution in the peptide of SEQ ID NO: 1 so that the sequence does not include "the sequence of 18 amino acids exhibiting the four sided structure of the present invention" was evaluated for the nucleic acid-introducing ability by the procedure described in Example 9. The fluorescent count was less than 10,000, and no nucleic acid-introducing ability was found.

### <Comparative Example 3>

Polylysine (average molecular weight: 11,000) was evaluated for its affinity for phosphatidyl serine by the procedure described in Example 20. It was then found that polylysine had no affinity for phosphatidyl serine. Polylysine was also evaluated for absorption in BSA solution by the procedure described in Example 4. The value measured was 1 or higher, and formation of aggregates was thus confirmed.

### <Comparative Example 4>

46 (Niidome, T et al., J. Biol. Chem., Vol. 272, 15307 (1997)) (the peptide of SEQ ID NO: 27) which is an amphipathic basic peptide having α-helix structure was evaluated for its affinity for phosphatidyl serine by the procedure described in Example 20. It was then found that this peptide had no affinity for phosphatidyl serine. This peptide was also evaluated for absorption in BSA solution by the procedure described in Example 4. The value measured was 1 or higher, and formation of aggregates was thus confirmed.

### <Comparative Example 5>

4₆ described in Comparative Example 4 (the peptide of SEQ ID NO: 27) was administered to a mouse by the procedure described in Example 26. The mouse died immediately after the administration.

It was estimated that this result reflected the situation that this peptide is an amphipathic basic peptide having α-helix structure which easily forms aggregates in blood and which exhibits serious toxicity upon administration to an animal.

### INDUSTRIAL APPLICABILITY OF THE INVENTION

The present invention is capable of providing a novel peptide chemically modified with PEG which is highly safe; which can be easily produced into a complex with a substance which binds to the peptide (enjoying excellent handling convenience), the thus produced complex exhibits excellent solubility; which can serve a vector with high selectivite and efficient introduction of the substance which binds to the peptide; and whose specific activity has not been compensated by the chemical modification with the PEG; as well as its production method.

The present invention is also capable of providing a complex of the peptide chemically modified with PEG and a substance which binds to the peptide, and its production method.

The present invention is also capable of providing a carrier modified with the peptide chemically modified with PEG, and its production method.

Typical merits of the present invention are as described below.

The peptide of the present invention is useful as a peptide vector since it has ability of binding to a nucleic acid and ability of introducing the nucleic acid into a cell.

Since the peptide takes α-helix structure only in the presence of a particular substance, it does not substantially form aggregates in serum and remains highly soluble.

In addition, when the peptide binds to a nucleic acid, the nucleic acid is stable since it is imparted with nuclease resistance.

Furthermore, the peptide has affinity for phosphatidyl serine, and therefore, it can selectively introduce the nucleic acid to the cell, tissue, or organ at the site where the so called immune response has taken place, for example, by inflammation, cell activation or cytotoxicity by immunocompetent cell, or apoptosis, the site where the cells have become malignantly transformed through abnormal cell division, the site where cytotoxicity of the cells constituting blood vessel have proceeded by the progress of blood coagulation or arterial sclerosis, the site where cytotoxic reaction has proceeded by super oxide, the site where cell activation and/or cytotoxic reaction has proceeded by a protease, and therefore, the nucleic acid can be administered at a reduced dose with reduced side effects.

The PEG-modified peptide of the present invention has the characters and merits as described above, and also, because of the chemical modification with PEG, it exhibits improved incorporation rate into the target cell of the genes and the drugs, improved pharmacological activity, and reduced toxicity.

## Claims

1. A peptide chemically modified with polyethylene glycol (PEG), including a sequence of 18 amino acids,
wherein
said sequence of 18 amino acids is constituted from alternately arranged two hydrophobic sides and two hydrophilic sides in α-helix structural model depicted by Edmundson wheel plots,
one of said hydrophobic sides comprises 5 to 7 amino acids and 80 mole% or more of this side comprises hydrophobic amino acids,
one of said hydrophilic sides comprises 5 or 6 amino acids, and 80 mole% or more of this side comprises hydrophilic amino acids, and 50 mole% or more of this side comprises an amino acid selected from the group consisting of arginine and lysine,
the other of said hydrophobic sides comprises 2 to 4 hydrophobic amino acids, and
the other of said hydrophilic sides comprises 3 to 5 amino acids and 80 mole% or more of this side comprises hydrophilic amino acids.

2. A peptide chemically modified with PEG according to claim 1 wherein said peptide comprises 20 or more amino acids in total; opposite ends of said peptide are N and C terminals; and any 18 consecutive amino acids in said peptide excluding the amino acids at opposite ends constitutes said sequence of 18 amino acids.

3. A peptide chemically modified with PEG according to claim 1 or 2 wherein the amino acids at the N and C terminals are each a hydrophilic amino acid.

4. A peptide chemically modified with PEG according to any one of claims 1 to 3 wherein said sequence of 18 amino acids is a sequence of any 18 consecutive amino acids in the following amino acid sequence:
X2-X3-X9-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36,
provided that,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
X3, X10, X12, X21, X28, and X30 are independently a hydrophobic amino acid, a neutral hydrophilic amino acid, or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid.

5. A peptide chemically modified with PEG according to any one of claims 1 to 4 wherein peptide moiety of said peptide chemically modified with PEG and including said sequence of 18 amino acids comprises the following amino acid sequence:
X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36-X37,
provided that
X1 and X37 are a hydrophilic amino acid,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
X3, X10, X12, X21, X28 and X30 are independently a hydrophobic amino acid, a neutral hydrophilic amino acid, or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid; and
wherein the sequence of amino acids X2 to X36 may include deletion, addition, insertion, or substitution as long as at least 18 amino acids are conserved in consecutive form.

6. A peptide chemically modified with PEG according to claim 5 wherein X1 to X37 are the following amino acids:
X1 is threonine,
X37 is serine,
X2, X5, X9, X20, X23, and X27 are independently arginine or lysine,
X3 and X21 are independently tyrosine, phenylalanine, serine, or arginine,
X4, X17, X22, and X35 are independently leucine,
X6, X15, X24, and X33 are independently leucine or isoleucine,
X7, X13, X25, and X31 are independently histidine or arginine,
X8 and X26 are independently proline,
X10 and X28 are independently serine, arginine, or leucine,
X11 and X29 are independently tryptophan or leucine,
X12 and X30 are independently valine, leucine, or serine,
X14 and X32 are independently glutamine, asparagine, or arginine,
X16 and X34 are independently alanine or arginine,
X18 is arginine, lysine, or serine,
X19 is leucine or threonine, and
X36 is arginine or serine; and
wherein the sequence of amino acids X2 to X36 may include deletion, addition, insertion, or substitution as long as at least 18 amino acids are conserved in consecutive form.

7. A peptide chemically modified with PEG according to any one of claims 1 to 6 wherein peptide moiety of said peptide chemically modified with PEG and including said sequence of 18 amino acids comprises any one of the amino acid sequences of SEQ ID NO: 1 to SEQ ID NO: 24.

8. A peptide chemically modified with PEG according to any one of claims 1 to 6 wherein peptide moiety of said peptide chemically modified with PEG and including said sequence of 18 amino acids comprises the amino acid sequence of SEQ ID NO: 16 or SEQ ID NO: 19.

9. A peptide chemically modified with PEG according to any one of claims 1 to 8 wherein PEG moiety of said peptide chemically modified with PEG and including said sequence of 18 amino acids has a molecular weight of about 200 Da to about 100,000 Da.

10. A complex comprising a peptide chemically modified with PEG and including a sequence of 18 amino acids, and a substance which binds to said peptide wherein
said sequence of 18 amino acids is constituted from alternately arranged two hydrophobic sides and two hydrophilic sides in α-helix structural model depicted by Edmundson wheel plots,
one of said hydrophobic sides comprises 5 to 7 amino acids and 80 mole% or more of this side comprises hydrophobic amino acids,
one of said hydrophilic sides comprises 5 or 6 amino acids, and 80 mole% or more of this side comprises hydrophilic amino acids, and 50 mole% or more of this side comprises an amino acid selected from the group consisting of arginine and lysine,
the other of said hydrophobic sides comprises 2 to 4 hydrophobic amino acids, and
the other of said hydrophilic sides comprises 3 to 5 amino acids and 80 mole% or more of this side comprises hydrophilic amino acids.

11. A complex according to claim 10 wherein said peptide comprises 20 or more amino acids in total; opposite ends of said peptide are N and C terminals; and any 18 consecutive amino acids in said peptide excluding the amino acids at opposite ends constitutes said sequence of 18 amino acids.

12. A complex according to claim 10 or 11 wherein the amino acids at the N and C terminals are each a hydrophilic amino acid.

13. A complex according to any one of claims 10 to 12 wherein said sequence of 18 amino acids is a sequence of any 18 consecutive amino acids in the following amino acid sequence:
X2-X3-X4-X5-X6-X7-X8-X9-X10-X11-X12-X13-X14-X15-X16-X17-X18-X19-X20-X21-X22-X23-X29-X25-X26-X27-X28-X29-X30-X31-X32-X33-X34-X35-X36,
provided that,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
X3, X10, X12, X21, X28, and X30 are independently a hydrophobic amino acid, a neutral hydrophilic amino acid, or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid.

14. A complex according to any one of claims 10 to 13 wherein peptide moiety of said peptide chemically modified with PEG and including said sequence of 18 amino acids comprises the following amino acid sequence:
X1-X2-X3-X4-X5-X6-X7-X8-X9-X20-X11-X12-X13-X14-X15-X16-X17-X18-X19-X20-X21-X22-X23-X24-X25-X26-X27-X28-X29-X30-X31-X32-X33-X34-X35-X36-X37,
provided that
X1 and X37 are a hydrophilic amino acid,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids out of the 5 amino acids are a hydrophobic amino acid,
X3, X10, X12, X21, X28 and X30 are independently a hydrophobic amino acid, a neutral hydrophilic amino acid, or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids out of the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid; and
wherein the sequence of amino acids X2 to X36 may include deletion, addition, insertion, or substitution as long as at least 18 amino acids are conserved in consecutive form.

15. A complex according to claim 14 wherein X1 to X37 are the following amino acids:
X1 is threonine,
X37 is serine,
X2, X5, X9, X20, X23, and X27 are independently arginine or lysine,
X3 and X21 are independently tyrosine, phenylalanine, serine, or arginine,
X4, X17, X22, and X35 are independently leucine,
X6, X15, X24, and X33 are independently leucine or isoleucine,
X7, X13, X25, and X31 are independently histidine or arginine,
X8 and X26 are independently proline,
X10 and X28 are independently serine, arginine, or leucine,
X11 and X29 are independently tryptophan or leucine,
X12 and X30 are independently valine, leucine, or serine,
X14 and X32 are independently glutamine, asparagine, or arginine,
X16 and X34 are independently alanine or arginine,
X18 is arginine, lysine, or serine,
X19 is leucine or threonine, and
X36 is arginine or serine; and
wherein the sequence of amino acids X2 to X36 may include deletion, addition, insertion, or substitution as long as at least 18 amino acids are conserved in consecutive form.

16. A complex according to any one of claims 10 to 15 wherein peptide moiety of said peptide chemically modified with PEG and including said sequence of 18 amino acids comprises any one of the amino acid sequences of SEQ ID NO: 1 to SEQ ID NO: 24.

17. A complex according to any one of claims 10 to 15 wherein peptide moiety of said peptide chemically modified with PEG and including said sequence of 18 amino acids comprises the amino acid sequence of SEQ ID NO: 16 or SEQ ID NO: 19.

18. A complex according to any one of claims 10 to 17 wherein said substance which binds to the peptide is a nucleic acid.

19. A complex according to any one of claims 10 to 18 wherein PEG moiety of said peptide chemically modified with PEG and including said sequence of 18 amino acids has a molecular weight of about 200 Da to about 100,000 Da.

20. A method for producing the peptide chemically modified with PEG of any one of claims 1 to 9 comprising the step of reacting a peptide comprising said sequence of 18 amino acids with activated polyethylene glycol.

21. A peptide chemically modified with polyethylene glycol (PEG) which is produced by the method of claim 20.

22. A method for producing the complex of any one of claims 10 to 19 comprising the steps of
a) reacting a peptide comprising said sequence of 18 amino acids with activated polyethylene glycol (PEG), and
b) reacting the peptide chemically modified with PEG that is obtained in said a) with a substance which binds to said peptide.

23. A method for producing the complex of any one of claims 10 to 19 comprising the steps of
a) reacting a peptide comprising said sequence of 18 amino acids with a substance which binds to said peptide, and
b) reacting the reaction product of said peptide and said substance which binds to said peptide with activated polyethylene glycol (PEG).

24. A complex of a peptide chemically modified with polyethylene glycol (PEG) and a substance which binds to said peptide, said complex being produced by the method of claim 22 or 23.

25. A carrier which is modified with the peptide chemically modified with PEG according to any one of claims 1 to 8.

26. A method for producing the carrier of claim 25 which is modified with the peptide chemically modified with PEG comprising the steps of
a) reacting a peptide comprising said sequence of 18 amino acids, or a peptide comprising said sequence of 18 amino acids and having cysteine attached to N or C terminal of the peptide, with activated PEG, and
b) reacting the reaction product of said a) with a carrier, or constructing a carrier by using the reaction product of said a) as a constituent.

27. A carrier which is modified with the peptide chemically modified with PEG, said carrier being produced by the method of claim 26.

28. A method for delivering a substance to the interior of a cell, said substance being bonded to or incorporated in the carrier of claim 25 that has been modified with the peptide chemically modified with PEG.


SEQUENCE LISTING

<110> Mochida Pharmaceutical Co.,Ltd.

<120> polyethyleneglycol modified peptide

<130> PCT196

<160> 30

<210> 1

<211> 37

<212> PRT

<213> Artificial Sequence

<220>

<223> Description of Artificial Sequence:Peptide

<400> 1

Thr Arg Tyr Leu Arg Ile His Pro Arg Ser Trp Val His Gln Ile Ala

1               5                   10                  15

Leu Arg Leu Arg Tyr Leu Arg Ile His Pro Arg Ser Trp Val His Gln

            20                  25                  30

Ile Ala Leu Arg Ser

        35

<210> 2

<211> 37

<212> PRT

<213> Artificial Sequence

<220> Description of Artificial Sequence:Peptide

<400> 2

Thr Arg Tyr Leu Arg Ile His Pro Arg Ser Trp Val Arg Gln Ile Ala

1               5                   10                  15

Leu Arg Leu Arg Tyr Leu Arg Ile His Pro Arg Ser Trp Val Arg Gln

            20                  25                  30

Ile Ala Leu Arg Ser

        35

<210> 3

<211> 37

<212> PRT

<213> Artificial Sequence

<220>

<223> Description of Artificial Sequence:Peptide

<400> 3

Thr Arg Tyr Leu Arg Ile His Pro Arg Ser Trp Val His Gln Ile Arg
1               5                   10                  15
Leu Arg Leu Arg Tyr Leu Arg Ile His Pro Arg Ser Trp Val His Gln
            20                  25                  30
Ile Arg Leu Arg Ser
        35

<210> 4
<211> 37
<212> PRT
<213> Artificial Sequence
<220>
<223> Description of Artificial Sequence:Peptide
<400> 4
Thr Arg Tyr Leu Arg Ile His Pro Arg Ser Trp Leu His Gln Ile Ala
1               5                   10                  15
Leu Arg Leu Arg Tyr Leu Arg Ile His Pro Arg Ser Trp Leu His Gln
            20                  25                  30
Ile Ala Leu Arg Ser
        35

<210> 5

<211> 37

<212> PRT

<213> Artificial Sequence

<220>

<223> Description of Artificial Sequence:Peptide

<400> 5

Thr Arg Phe Leu Arg Ile His Pro Arg Ser Trp Val His Gln Ile Ala

1               5                   10                  15

Leu Arg Leu Arg Phe Leu Arg Ile His Pro Arg Ser Trp Val His Gln

            20                  25                  30

Ile Ala Leu Arg Ser

        35

<210> 6

<211> 37

<212> PRT

<213> Artificial Sequence

<220>

<223> Description of Artificial Sequence:Peptide

<400> 6

Thr Arg Tyr Leu Arg Ile His Pro Arg Ser Trp Val His Asn Ile Ala
1               5                   10                  15
Leu Arg Leu Arg Tyr Leu Arg Ile His Pro Arg Ser Trp Val His Asn
            20                  25                  30
Ile Ala Leu Arg Ser
        35

<210> 7
<211> 37
<212> PRT
<213> Artificial Sequence
<220>
<223> Description of Artificial Sequence:Peptide
<400> 7
Thr Arg Tyr Leu Arg Ile His Pro Arg Ser Trp Val His Gln Ile Ala
1               5                   10                  15
Leu Lys Leu Lys Tyr Leu Arg Ile His Pro Arg Ser Trp Val His Gln
            20                  25                  30
Ile Ala Leu Arg Ser
        35

<210> 8

<211> 37

<212> PRT

<213> Artificial Sequence

<220>

<223> Description of Artificial Sequence:Peptide

<400> 8

Thr Arg Tyr Leu Arg Ile His Pro Lys Ser Trp Val His Gln Ile Ala

1               5                   10                  15

Leu Arg Leu Arg Tyr Leu Lys Ile His Pro Arg Ser Trp Val His Gln

            20                  25                  30

Ile Ala Leu Arg Ser

        35

<210> 9

<211> 37

<212> PRT

<213> Artificial Sequence

<220>

<223> Description of Artificial Sequence:Peptide

<400> 9

Thr Arg Tyr Leu Arg Ile His Pro Arg Ser Trp Val His Gln Ile Ala
1               5                   10                  15
Leu Arg Thr Arg Tyr Leu Arg Ile His Pro Arg Ser Trp Val His Gln
            20                  25                  30
Ile Ala Leu Arg Ser
        35

<210> 10
<211> 37
<212> PRT
<213> Artificial Sequence
<220>
<223> Description of Artificial Sequence:Peptide
<400> 10
Thr Arg Ser Leu Arg Ile His Pro Arg Ser Trp Val His Gln Ile Ala
1               5                   10                  15
Leu Arg Leu Arg Ser Leu Arg Ile His Pro Arg Ser Trp Val His Gln
            20                  25                  30
Ile Ala Leu Arg Ser
        35

<210> 11

<211> 37

<212> PRT

<213> Artificial Sequence

<220>

<223> Description of Artificial Sequence:Peptide

<400> 11

Thr Arg Tyr Leu Arg Ile His Pro Arg Leu Trp Val His Gln Ile Ala

1               5                   10                  15

Leu Arg Leu Arg Tyr Leu Arg Ile His Pro Arg Leu Trp Val His Gln

            20                  25                  30

Ile Ala Leu Arg Ser

        35

<210> 12

<211> 37

<212> PRT

<213> Artificial Sequence

<220>

<223> Description of Artificial Sequence:Peptide

<400> 12

Thr Arg Tyr Leu Arg Ile His Pro Arg Ser Trp Val His Gln Ile Ala
1               5                   10                  15
Leu Ser Leu Arg Tyr Leu Arg Ile His Pro Arg Ser Trp Val His Gln
            20                  25                  30
Ile Ala Leu Ser Ser
        35

<210> 13
<211> 37
<212> PRT
<213> Artificial Sequence
<220>
<223> Description of Artificial Sequence:Peptide
<400> 13
Thr Arg Tyr Leu Arg Ile His Pro Arg Ser Leu Val His Gln Ile Ala
1               5                   10                  15
Leu Arg Leu Arg Tyr Leu Arg Ile His Pro Arg Ser Leu Val His Gln
            20                  25                  30
Ile Ala Leu Arg Ser
        35

<210> 14

<211> 37

<212> PRT

<213> Artificial Sequence

<220>

<223> Description of Artificial Sequence:Peptide

<400> 14

Thr Arg Tyr Leu Arg Ile His Pro Arg Ser Trp Ser His Gln Ile Ala

1               5                   10                  15

Leu Arg Leu Arg Tyr Leu Arg Ile His Pro Arg Ser Trp Ser His Gln

            20                  25                  30

Ile Ala Leu Arg Ser

        35

<210> 15

<211> 37

<212> PRT

<213> Artificial Sequence

<220>

<223> Description of Artificial Sequence:Peptide

<400> 15

Thr Arg Tyr Leu Arg Ile Arg Pro Arg Ser Trp Val Arg Gln Ile Ala
1               5                   10                  15
Leu Arg Leu Arg Tyr Leu Arg Ile Arg Pro Arg Ser Trp Val Arg Gln
            20                  25                  30
Ile Ala Leu Arg Ser
        35

<210> 16
<211> 37
<212> PRT
<213> Artificial Sequence
<220>
<223> Description of Artificial Sequence:Peptide
<400> 16
Thr Arg Tyr Leu Arg Leu His Pro Arg Ser Trp Val His Gln Leu Ala
1               5                   10                  15
Leu Arg Leu Arg Tyr Leu Arg Leu His Pro Arg Ser Trp Val His Gln
            20                  25                  30
Leu Ala Leu Arg Ser
        35

<210> 17

<211> 37

<212> PRT

<213> Artificial Sequence

<220>

<223> Description of Artificial Sequence:Peptide

<400> 17

Thr Arg Ser Leu Arg Ile His Pro Arg Leu Trp Val His Gln Ile Ala

1               5                   10                  15

Leu Arg Leu Arg Ser Leu Arg Ile His Pro Arg Leu Trp Val His Gln

            20                  25                  30

Ile Ala Leu Arg Ser

        35

<210> 18

<211> 37

<212> PRT

<213> Artificial Sequence

<220>

<223> Description of Artificial Sequence:Peptide

<400> 18

Thr Arg Tyr Leu Arg Ile His Pro Arg Ser Trp Leu His Gln Ile Ala
1               5                   10                  15
Leu Arg Thr Arg Tyr Leu Arg Ile His Pro Arg Ser Trp Leu His Gln
            20                  25                  30
Ile Ala Leu Arg Ser
        35

<210> 19
<211> 37
<212> PRT
<213> Artificial Sequence
<220>
<223> Description of Artificial Sequence:Peptide
<400> 19
Thr Arg Tyr Leu Arg Leu His Pro Arg Ser Trp Leu His Gln Leu Ala
1               5                   10                  15
Leu Arg Leu Arg Tyr Leu Arg Leu His Pro Arg Ser Trp Leu His Gln
            20                  25                  30
Leu Ala Leu Arg Ser
        35

<210> 20

<211> 37

<212> PRT

<213> Artificial Sequence

<220>

<223> Description of Artificial Sequence:Peptide

<400> 20

Thr Arg Tyr Leu Arg Leu His Pro Arg Ser Trp Val His Gln Leu Ala

1               5                   10                  15

Leu Arg Thr Arg Tyr Leu Arg Leu His Pro Arg Ser Trp Val His Gln

            20                  25                  30

Leu Ala Leu Arg Ser

        35

<210> 21

<211> 37

<212> PRT

<213> Artificial Sequence

<220>

<223> Description of Artificial Sequence:Peptide

<400> 21

Thr Lys Tyr Leu Lys Ile His Pro Lys Ser Trp Val His Gln Ile Ala
1               5                   10                  15
Leu Arg Leu Lys Tyr Leu Lys Ile His Pro Lys Ser Trp Val His Gln
            20                  25                  30
Ile Ala Leu Arg Ser
        35

<210> 22
<211> 37
<212> PRT
<213> Artificial Sequence
<220>
<223> Description of Artificial Sequence:Peptide
<400> 22
Thr Arg Arg Leu Arg Ile His Pro Arg Arg Trp Val His Arg Ile Ala
1               5                   10                  15
Leu Arg Leu Arg Arg Leu Arg Ile His Pro Arg Arg Trp Val His Arg
            20                  25                  30
Ile Ala Leu Arg Ser
        35

<210> 23

<211> 31

<212> PRT

<213> Artificial Sequence

<220>

<223> Description of Artificial Sequence:Peptide

<400> 23

Thr Ile His Pro Arg Ser Trp Val His Gln Ile Ala Leu Arg Leu Arg

1               5                   10                  15

Tyr Leu Arg Ile His Pro Arg Ser Trp Val His Gln Ile Ala Ser

            20                  25                  30

<210> 24

<211> 20

<212> PRT

<213> Artificial Sequence

<220>

<223> Description of Artificial Sequence:Peptide

<400> 24

Thr Leu Arg Tyr Leu Arg Ile His Pro Arg Ser Trp Leu His Gln Ile

1               5                   10                  15

Ala Leu Arg Ser

            20

<210> 25

<211> 37

<212> PRT

<213> Artificial Sequence

<220>

<223> Description of Artificial Sequence:Peptide

<400> 25

Thr Ile Arg Tyr Arg Pro Ser His Gln Ile Arg Leu Arg Ala Val Leu

1               5                   10                  15

His Leu Trp Ile Arg Tyr Arg Pro Ser His Gln Ile Arg Leu Arg Ala

            20                  25                  30

Val Leu His Trp Ser

        35

<210> 26

<211> 37

<212> PRT

<213> Artificial Sequence

<220>

<223> Description of Artificial Sequence:Peptide

<400> 26

Thr Arg Tyr Leu Arg Ile His Pro Arg Ser Trp Val Leu Gln Ile Ala

1               5                   10                  15

Leu Arg Leu Arg Tyr Leu Arg Ile His Pro Arg Ser Trp Val Leu Gln

            20                  25                  30

Ile Ala Leu Arg Ser

        35

<210> 27

<211> 24

<212> PRT

<213> Artificial Sequence

<220>

<223> Description of Artificial Sequence:Peptide

<400> 27

Leu Ala Arg Leu Leu Ala Arg Leu Leu Ala Arg Leu Leu Arg Ala Leu

1               5                   10                  15

Leu Arg Ala Leu Leu Arg Ala Leu

            20

<210> 28

<211> 111

<212> DNA

<213> Artificial Sequence

<220>

<223> Description of Artificial Sequence:DNA

<400> 28

actcgttatc ttcgcattca tcctcgaagt tgggttcacc aaatagctct gagactacgg 60

tacttacgaa ttcacccacg tagctgggtt caccaaatag ctctgcgttc t 111

<210> 29

<211> 111

<212> DNA

<213> Artificial Sequence

<220>

<223> Description of Artificial Sequence:DNA

<400> 29

actcgttatc ttcgccttca tcctcgaagt tgggttcacc aactagctct gagactacgg 60

tacttacgac ttcacccacg tagctgggtt caccaactag ctctgcgttc t 111

<210> 30

<211> 111

<212> DNA

<213> Artificial Sequence

<220>

<223> Description of Artificial Sequence:DNA

<400> 30

actcgttatc ttcgccttca tcctcgaagt tggcttcacc aactagctct gagactacgg 60

tacttacgac ttcacccacg tagctggctt caccaactag ctctgcgttc t 111